# EUROPEAN PATENT APPLICATION

(11) **EP 3 821 946 A1**
(43) Date of publication of application: **19.05.2021**
(21) Application number: 19208560.3
(22) Date of filing: 12.11.2019
(51) Int. Cl.: A61P 11/00, C07K 16/28, A61K 39/00

(54) **ANTI-CLAUDIN-1 MONOCLONAL ANTIBODIES FOR THE PREVENTION AND TREATMENT OF FIBROTIC DISEASES**

(71) Applicant: Université de Strasbourg, 67000 Strasbourg (FR); INSERM (Institut National de la Santé et de la Recherche Médicale), 75013 Paris (FR)
(72) Inventor: SAVIANO, Antonio, 67000 Strasbourg (FR); BAUMERT, Thomas, 79100 Freiburg (DE)
(74) Representative: Plasseraud IP

(57) **Abstract**

The present invention concerns the use of anti-Claudin-1 monoclonal antibodies, and pharmaceutical compositions thereof, for the prevention and/or treatment of a fibrotic disease in a patient, in particular pulmonary fibrosis, kidney fibrosis or skin fibrosis. Methods of preventing and/or treating pulmonary fibrosis kidney fibrosis or skin fibrosis by administration of such a monoclonal antibody, or a pharmaceutical composition thereof, are also described.

## Description

### Background of the Invention

Fibrotic disease is characterized by excessive deposition of fibrous connective tissue (a process called fibrosis) that can lead to progressive deterioration in the normal structure and function of organs and tissues of the body. Fibrosis is defined by the overgrowth, hardening and/or scarring of a tissue or organ. It is attributed to excessive accumulation of components of the extracellular matrix (ECM), such as collagen and fibronectin (Wynn et al., Nature Medicine, 2012, 18: 1028-1040), in and around inflamed or damaged tissue, which can lead to permanent scarring, organ malfunction and ultimately death. Fibrosis is the final, common pathological outcome of many chronic inflammatory reactions induced by a variety of stimuli including persistent infections, genetic disorders, autoimmune reactions, allergic response, chemical insults, radiations, and tissue injury. Fibrosis can occur in nearly every organ or tissue of the body, more often in the heart, lung, kidney, liver and skin (Rockey et al., N. Engl. J. Med., 2015, 372: 1138-1149) and less frequently in other tissues or organs such as the pancreas, intestine, eye (Wynn, J. Pathol., 2008, 214: 199-210), nerve system (Kawano et al., Cell Tissue Res., 2012, 349: 169-180), mediastinum (Parish and Rosenow, Semin. Respir. Crit. Care Med., 2002, 23: 135-143), retroperitoneum (Caiafa et al., Radiographics, 2013, 33: 535-552), joint and tendon.

Human fibrotic diseases have a poor prognosis comparable with end-stage cancer. They represent an increasing cause of morbidity and mortality worldwide. Since fibrosis is a predominant feature of the pathology of a wide range of diseases across multiple organ systems, fibrotic disorders have been estimated to contribute to about 45% of all-cause mortality in the United States (Wynn, Nature Rev. Immunol., 2004, 4: 583-594). The major health problem associated with fibrotic diseases is also due to our incomplete understanding of the fibrotic process pathogenesis, the marked heterogeneity in the etiologies and clinical manifestations of fibrotic disorders, the absence of appropriate and fully validated biomarkers, and most importantly, the current void of effective disease-modifying therapeutic agents. Indeed, at present, there are only two recently approved drugs specifically indicated for the treatment of fibrotic disease.

In light of the economic burden of patients with fibrotic disease worldwide, and in view of the limited therapeutic arsenal, novel strategies to prevent and/or treat fibrosis are urgently needed.

### Summary of the Invention

The present invention relates to systems and strategies for the prevention and/or treatment of fibrotic diseases, in particular pulmonary fibrosis, renal fibrosis and skin fibrosis. In particular, the present invention is directed to the use of anti-Claudin-1 antibodies for preventing and/or treating pulmonary fibrosis, renal fibrosis or skin fibrosis. Indeed, the present Inventors have shown that, *in vivo,* an anti-Claudin-1 monoclonal antibody specifically binds its target in the most important organs, and in particular in the lungs, the kidneys and the skin, without any detectable toxicity. Furthermore, using a state-of-the-art mouse model considered as the most important preclinical model of pulmonary fibrosis, they demonstrated that the anti-Claudin-1 monoclonal antibody prevents the formation of lung fibrosis without affecting overall survival or body weight and reduces fibrosis levels in lungs without detectable adverse effects. They have also shown that the anti-Claudin-1 monoclonal antibody binds to Claudin-1 expressed on kidney and lung cancer cells, and reverses fibrosis-related poor-prognosis of a clinical gene-signature in lung cells.

Consequently, in one aspect, the present invention provides an anti-Claudin-1 antibody, or a biologically active fragment thereof, for use in the prevention or treatment of a fibrotic disease selected from the group consisting of pulmonary fibrosis, renal fibrosis, and skin fibrosis.

In certain embodiments, the pulmonary fibrosis is selected from the group consisting of idiopathic pulmonary fibrosis (IPF), idiopatic nonspecific interstitial pneumonitis (NSIP), cryptogenic organizing pneumonia (COP), Hamman-Rich syndrome, lymphocytic interstitial pneumonitis (LIP), respiratory bronchiolitis interstitial lung disease, desquamative interstitial pneumonitis or idiopathic lymphoid interstitial pneumonia, and idiopathic pleuroparenchymal fibroelastosis.

In certain embodiments, the pulmonary fibrosis is associated with chronic obstructive pulmonary disease.

In certain embodiments, the fibrotic disease is pulmonary fibrosis and the anti-Claudin-1 antibody, or the biologically active fragment thereof, is administered in combination with at least one therapeutic agent selected from the group consisting of corticosteroids, anti-fibrotic agents, pirfenidone, nintedanib, and anti-acid drugs, and/or with a therapeutic procedure selected from the group consisting of lung transplantation, hyperbaric oxygen therapy and pulmonary rehabilitation.

In other embodiments, the renal fibrosis is renal interstitial fibrosis or glomerulosclerosis.

In certain embodiments, the renal fibrosis is associated with chronic kidney disease.

In certain embodiments, the fibrotic disease is renal fibrosis and the anti-Claudin-1 antibody, or the biologically active fragment thereof, is administered in combination with at least one therapeutic agent selected from the group consisting of anti-hypertensive drugs 1,25-dihydroxyvitamin D3, erythropoietin, angiotensin converting enzyme inhibitors, angiotensin II receptor antagonists AST-120, and calcium polystyrene sulfonate, and/or with one therapeutic procedure selected from the group consisting of dialysis and kidney transplantation.

In certain embodiments, the skin fibrosis is associated with a medical condition selected from the group consisting of scleroderma in both localized (morphea, linear scleroderma) and systemic forms, graft-versus-host disease (GVHD), nephrogenic fibrosing dermopathy, mixed connective tissue disease, scleredema, scleromyxedema, eosinophilic fasciitis, chromoblastomycosis, hypertrophic scars and keloids. In certain embodiments, the skin fibrosis is induced by a medical intervention (*e.g.,* radiotherapy), by environmental or professional exposures to chemicals (*e.g.,* in eosinophilia-myalgia syndrome induced by L-tryptophan); and exposure to certain physical agents (physical trauma, surgical injury, heat or ice skin burns).

In certain embodiments, the fibrotic disease is skin fibrosis and the anti-Claudin-1 antibody, or the biologically active fragment thereof, is administered in combination with at least one therapeutic agent selected from the group consisting methotrexate, mycophenolyate, mofetil, cyclophosphamide, cyclosporine, tocilizumab, rituximab, and fresolimumab and/or with one therapeutic procedure (e.g., ultraviolet radiation).

In certain embodiments, the anti-Claudin-1 antibody used in the prevention or treatment of pulmonary fibrosis, renal fibrosis or skin fibrosis is a monoclonal antibody.

In certain embodiments, the anti-Claudin-1 monoclonal antibody has the same epitope as a monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

In certain embodiments, the epitope is strongly dependent on the conservation of the conserved motif W(30)-GLW(51)-C(54)-C(64) in Claudin-1 first extracellular loop.

In other embodiments, the anti-Claudin-1 antibody is a monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

In other embodiments, the anti-Claudin-1 antibody is a monoclonal antibody comprising the six complementary determining regions (CDRs) of a monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

In certain embodiments, the anti-Claudin-1 antibody used in the prevention or treatment of pulmonary fibrosis, renal fibrosis or dermal fibrosis is humanized.

The humanized anti-Claudin-1 antibody may be a monoclonal antibody comprising all of the six CDRs of monoclonal antibody OM-7D3-B3 secreted by hybridoma cell line deposited under Accession Number DSM ACC2938, wherein the variable heavy chain of OM-7D3-B3 consists of amino acid sequence SEQ ID NO: 1 and the variable light chain of OM-7D3-B3 consists of amino acid sequence SEQ ID NO: 2, said humanized anti-Claudin-1 monoclonal antibody further comprising:
a) at least one antibody variable heavy chain (VH) consisting of the amino acid sequence of SEQ ID NO: 3 or SEQ ID NO: 4 or SEQ ID NO: 5, or
b) at least one antibody variable light chain (VL) consisting of the amino acid sequence of SEQ ID NO: 6 or SEQ ID NO: 7 or SEQ ID NO: 8.

For example, the humanized anti-Claudin-1 monoclonal antibody may comprise:
a) two antibody variable heavy chains (VH), both variable heavy chains consisting of the amino acid sequence of SEQ ID NO: 3 or SEQ ID NO: 4 or SEQ ID NO: 5, or
b) two antibody variable light chains (VL), both variable light chains consisting of the amino acid sequence of SEQ ID NO: 6 or SEQ ID NO: 7 or SEQ ID NO: 8.

For example, the humanized anti-Claudin-1 monoclonal antibody may comprise:
1) two antibody variable heavy chains (VH) consisting of the amino acid sequence SEQ ID NO: 3, and two antibody variable light chains (VL) consisting of the amino acid sequence SEQ ID NO: 6 [*H3L3*]; or
2) two antibody variable heavy chains (VH) consisting of the amino acid sequence SEQ ID NO: 3, and two antibody variable light chains (VL) consisting of the amino acid sequence SEQ ID NO: 7 [*H3L1*]*;* or
3) two antibody variable heavy chains (VH) consisting of the amino acid sequence SEQ ID NO: 3, and two antibody variable light chains (VL) consisting of the amino acid sequence SEQ ID NO: 8 [*H3L2*]; or
4) two antibody variable heavy chains (VH) consisting of the amino acid sequence SEQ ID NO: 4, and two antibody variable light chains (VL) consisting of the amino acid sequence SEQ ID NO: 6 [*H1L3*]*;* or
5) two antibody variable heavy chains (VH) consisting of the amino acid sequence SEQ ID NO: 4, and two antibody variable light chains (VL) consisting of the amino acid sequence SEQ ID NO: 7 [*H1L1*]*;* or
6) two antibody variable heavy chains (VH) consisting of the amino acid sequence SEQ ID NO: 4, and two antibody variable light chains (VL) consisting of the amino acid sequence SEQ ID NO: 8 [*H1L2*]*;* or
7) two antibody variable heavy chains (VH) consisting of the amino acid sequence SEQ ID NO: 5, and two antibody variable light chains (VL) consisting of the amino acid sequence SEQ ID NO: 6 [*H2L3*]; or
8) two antibody variable heavy chains (VH) consisting of the amino acid sequence SEQ ID NO: 5, and two antibody variable light chains (VL) consisting of the amino acid sequence SEQ ID NO: 7 [*H2L1*]*;* or
9) two antibody variable heavy chains (VH) consisting of the amino acid sequence SEQ ID NO: 5, and two antibody variable light chains (VL) consisting of the amino acid sequence SEQ ID NO: 8 [*H2L2*].

In certain embodiments, the humanized anti-Claudin-1 monoclonal antibody is a full antibody having an isotype selected from the group consisting of IgG1, IgG2, IgG3, and IgG4.

The present invention also provides a pharmaceutical composition comprising an effective amount of an anti-Claudin-1 antibody, or a biologically active fragment thereof, and at least one pharmaceutically acceptable carrier or excipient, for use in the prevention or treatment of a fibrotic disease selected from pulmonary fibrosis, renal fibrosis, and skin fibrosis in a subject.

In such a use of pharmaceutical composition, the pulmonary fibrosis, the renal fibrosis and the skin fibrosis may be as defined above; and the anti-Claudin-1 antibody, or biologically active fragment thereof, may be as defined above.

The present invention further provides a method for preventing or treating a pulmonary fibrosis or renal fibrosis or skin fibrosis in a subject, the method comprising administering to the subject a therapeutically efficient amount of an anti-Claudin-1 antibody, or a biologically active fragment thereof. In such a method of prevention and/or treatment, the pulmonary fibrosis, the renal fibrosis, and the dermal fibrosis may be as defined above; and the anti-Claudin-1 antibody, or biologically active fragment thereof, may be as defined above.

These and other objects, advantages and features of the present invention will become apparent to those of ordinary skill in the art having read the following detailed description of the preferred embodiments.

### Brief Description of the Drawing

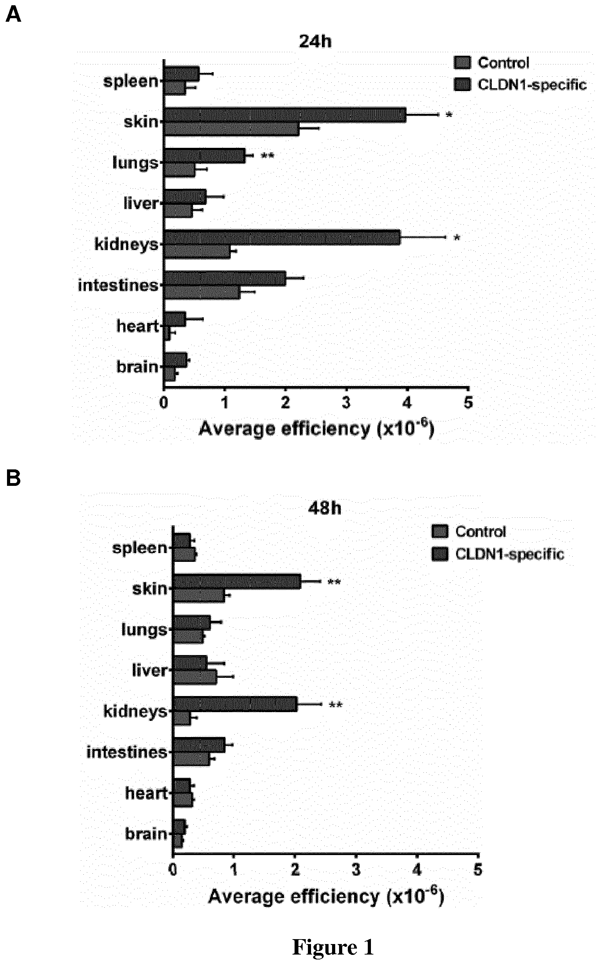

**Figure 1****. Biodistribution of Anti-CLDNl mAb in Mice.** Mice (n=6 per group/time point) were injected with 500 µg of Alexa fluor 750-control mAbs (grey bars) or CLDN1-specific mAbs (blue bars) and sacrificed at (**A**) 24 hours or (**B**) 48 hours following injection. The specific fluorescence was detected in each indicated organ using an IVIS Lumina 50 device with specific filter sets and expressed as average efficiency. The horizontal line indicates the mean. The results show the mean ± s.e.m. from 6 mice. **p* < 0.05, **p < 0.01 (Mann-Whitney test).

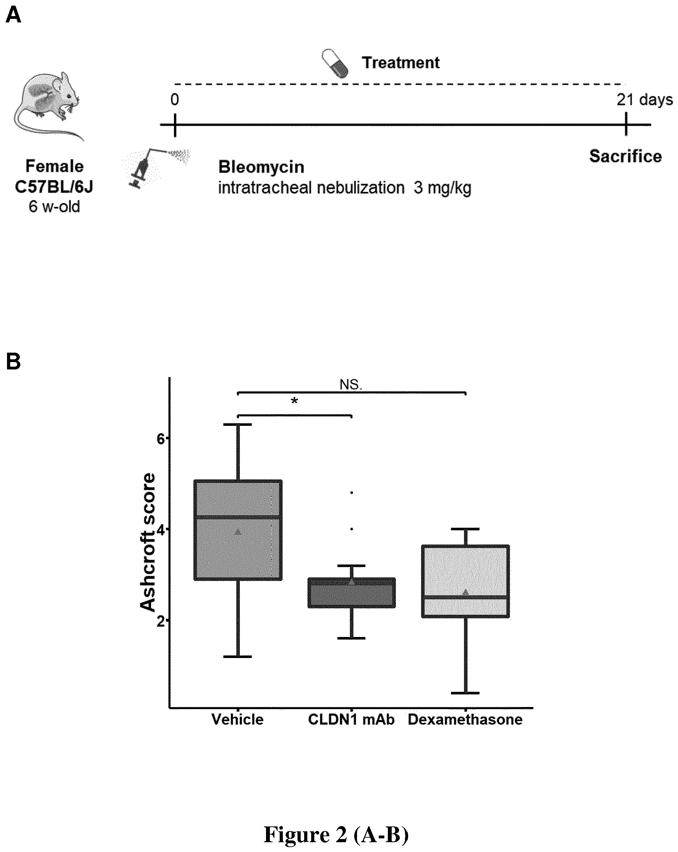

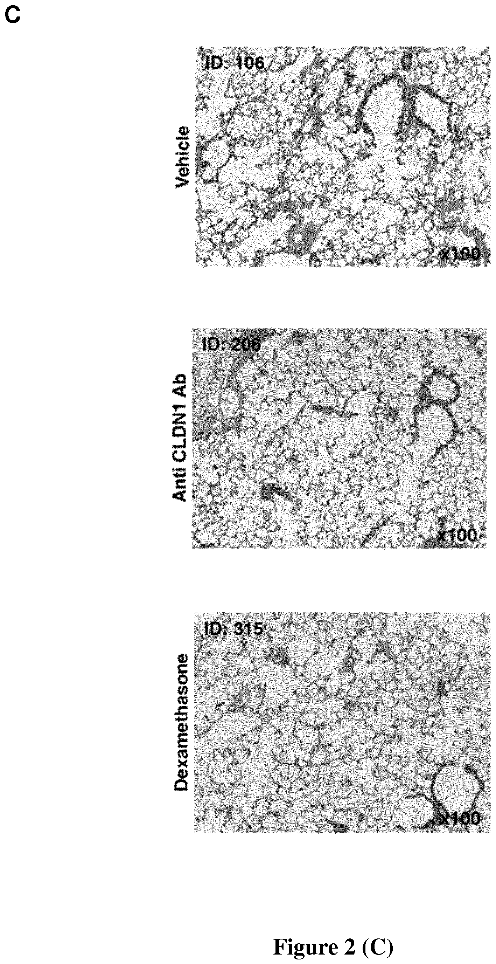

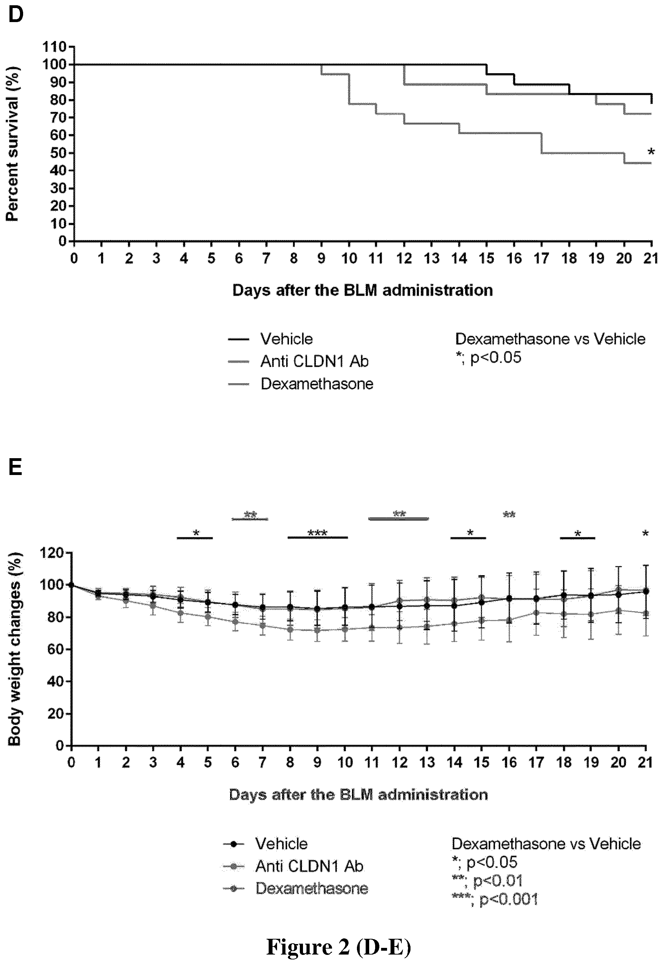

**Figure 2****. Anti-CLDNl mAb significantly Reduces Lung Fibrosis in a State-of-the-art Mouse Model for Prevention and Early Treatment of Pulmonary Fibrosis.** (**A**) Prevention and early treatment study design. Six-weeks old female C57BL/6J mice underwent intratracheal bleomycin nebulization (3 mg/kg) to induce pulmonary fibrosis and were randomized to receive vehicle, CLDN1-specific mAb and dexamethasone (positive control) for 21 days. Each group included 18 mice. (**B**) The Ashcroft fibrosis score was significantly reduced in the CLDN1 mAb group compared to the Vehicle group. Data are represented as mean (triangle), median (line), 1^{st} and 3^{rd} quartile (bottom and top of the box). (**C**) Trichromic masson staining of one representative mouse lung per treatment group. (**D**) Kaplan-Meier curves showing that the Dexamethasone treated mice had significant lower survival compared to the Vehicle group. (**E**) In the Dexamethasone group, a significant reduction of mouse body weight was observed during the experiment. **p* < 0.05, **p < 0.01, *** p<0.001, N.S.= not significant.

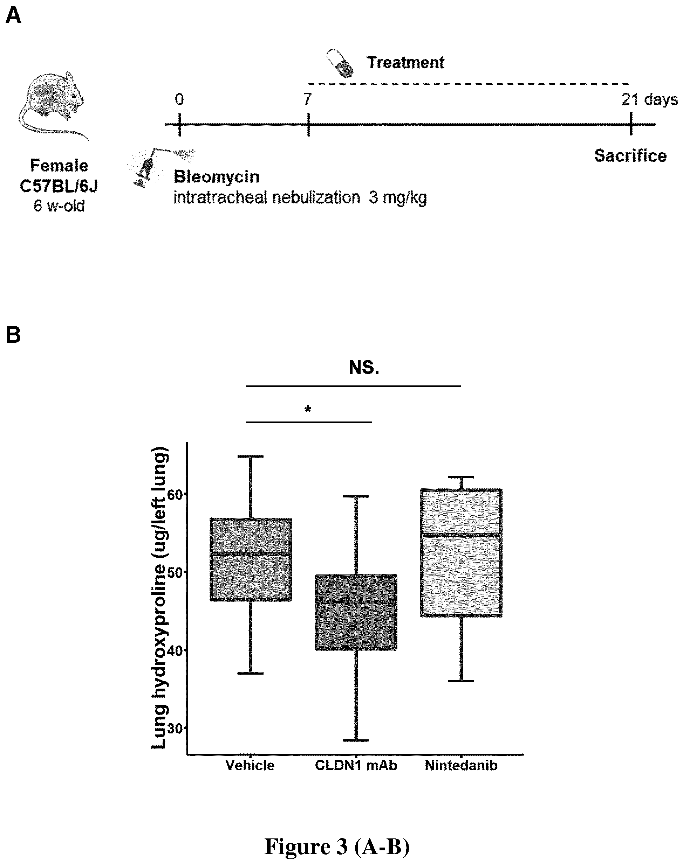

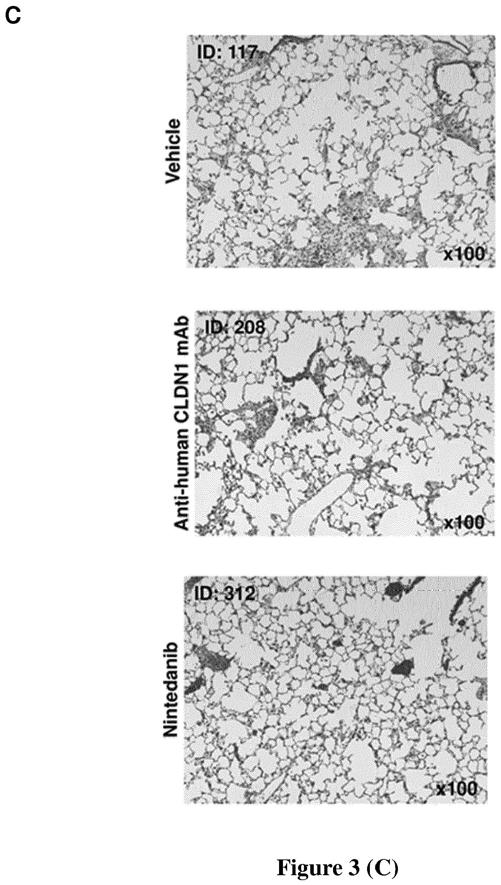

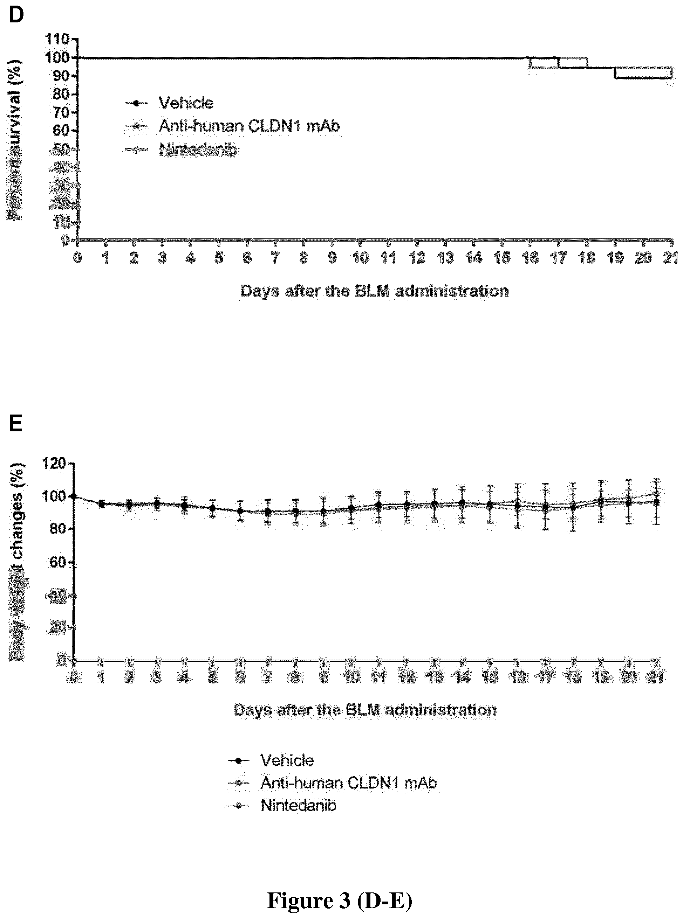

**Figure 3****. Anti-CLDNl mAb Reduces Lung Fibrosis in a State-of-the-art Mouse Model for Late Treatment of Pulmonary Fibrosis.** (**A**) Late treatment study design. Six-weeks old female C57BL/6J mice underwent intratracheal bleomycin nebulization (3 mg/kg) to induce pulmonary fibrosis and were randomized to receive vehicle, anti-human CLDN1-specific mAb or Nintedanib (positive control) from day 7 to day 21. Each group included 18 mice. (**B**) The lung hydroxyproline levels was significantly reduced in the CLDN1 mAb group. Body weight was not different between the two treatment groups. Data are represented as mean (triangle), median (line), 1^{st} and 3^{rd} quartile (bottom and top of the box). (**C**) Trichromic masson staining of one representative mouse lung per treatment group. (**D**) Kaplan-Meier curves showing no difference between the groups. (**E**) No significant reduction of mouse body weight was observed during the experiment in all the groups. **p* < 0.05, N.S.= not significant.

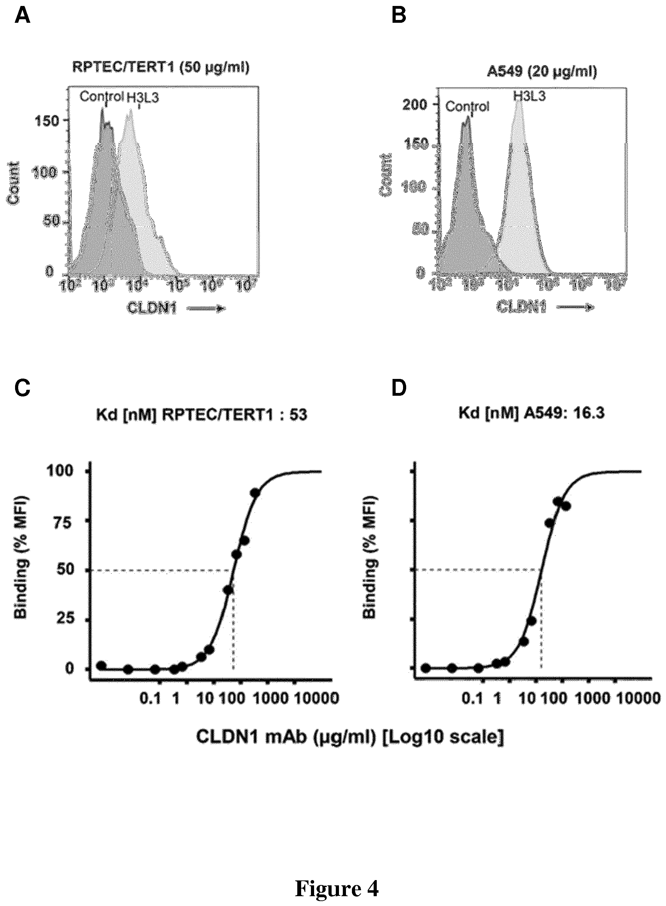

**Figure 4****. Binding Properties of Humanized CLDN1-specific MAbs Targeting CLDN1 Expressed on Kidney RPTEC/TERT1 and Lung A549 Cell Lines.** Cells were incubated with increasing concentrations of humanized H3L3 CLDN1-specific mAb, as indicated. Representative histograms show binding of humanized H3L3 CLDN1-specific mAb at the saturation point for each specific cells line: (**A**) **RPTEC/TERT1** (50 µg/mL) and (**B**) A549 (20 µg/mL). Binding was measured by flow cytometry after incubation with PE-labelled anti-human and analyzed with Cytoflex and FlowJo V10. The binding constants K_{d} of the interaction between the humanized H3L3 CLDN1-specific mAb and CLDN1 expressed by (**C**) **RPTEC/TERT1** (50 µg/mL) and (**D**) A549 (20 µg/mL) cells were determined by applying the Michaelis-Menten mathematical model in R 3.5.1 using the PE median fluorescence intensity (MFI), respectively. The graphs show binding of humanized H3L3 CLDN1-specific mAb until the saturation point for each specific cell line.

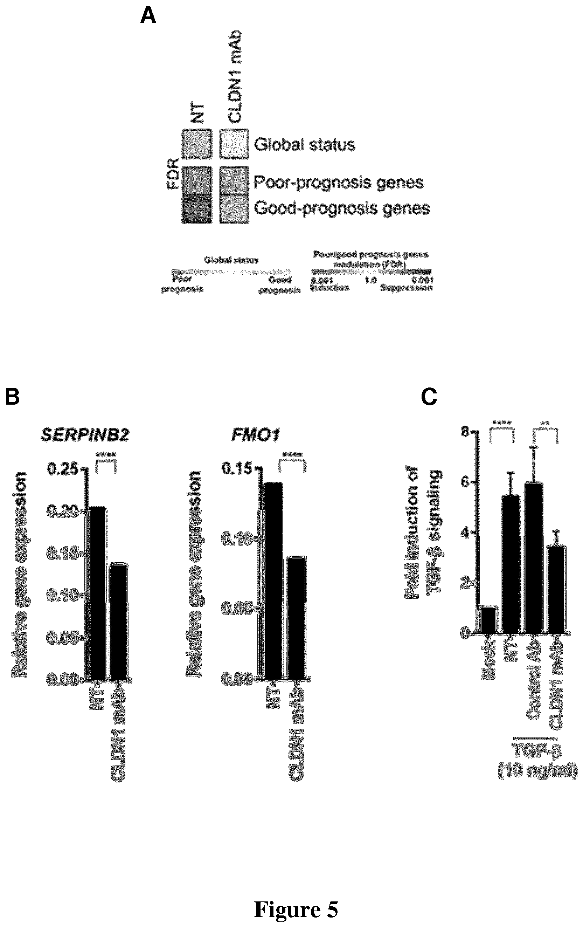

**Figure 5****. CLDN1-specific MAbs Reverses PLS and Reduces TGF-β Signaling in A549 Cells.** (**A**) A549 cells were cultured for 24 hours with or without TGF-β (5 ng/mL) and CLDN1 mAb (20 µg/mL) treatment for 24 hours. RNA was extracted and PLS gene expression was measuring using nCounter Nanostring technology and was assessed quantitatively by Gene Set Enrichment Analysis (GSEA). The heatmaps show: PLS status as poor (orange) or good (green) prognosis; (bottom) the significance of induction (red) or suppression (blue) of PLS poor- or good-prognosis genes. (**B**) Relative gene expression values showing the mean and standard error of mean of two poor-prognosis, differentially expressed genes *(SERPINB2* and *FMO1)* modulated by CLDN1-specific mAb treatment (n=3, fold change -1.7, ****p-value < 0.0001). NT: No treatment. (**C**) 4549 cells were transfected with TGF-β signaling reporter plasmid pGL4.48[luc2P/SBE/Hygro] (Promega) and treated with control or CLDN1-specific mAb (50 µg/mL) for 3 hours at 37°C. Cells were stimulated with medium containing TGF-β (10 ng/mL) for 3 hours at 37°C. Fold changes showing the mean and standard error of mean were calculated from luminescence intensities normalized to the mock samples (n=6, CLND1 mAb n=5, **** p-value < 0.0001, ** p-value < 0.01).

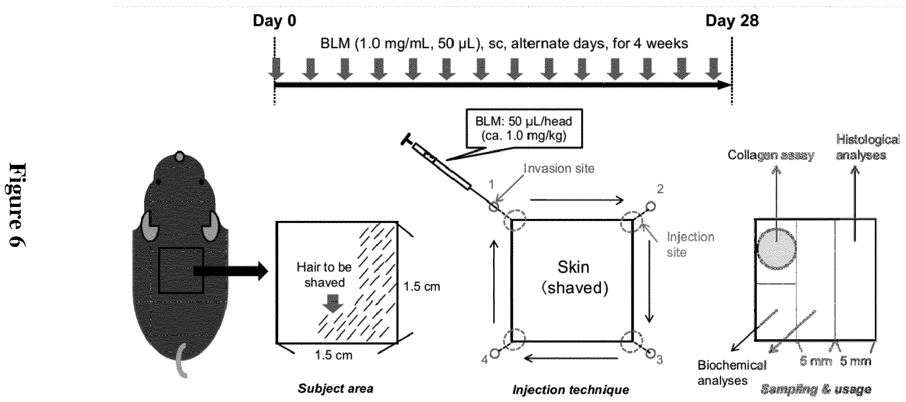

**Figure 6****. Bleomycin-induced Skin Fibrosis Model.** An area of 1.5x1.5 cm of the mouse skin was shaved and bleomycin (BLM) 1.0 mg/kg was administrered at the four corners s.c. alternate days for 4 weeks. The mouse was sacrificed and the skin area was sampled for collagen assay, histological and biochemical analysis.

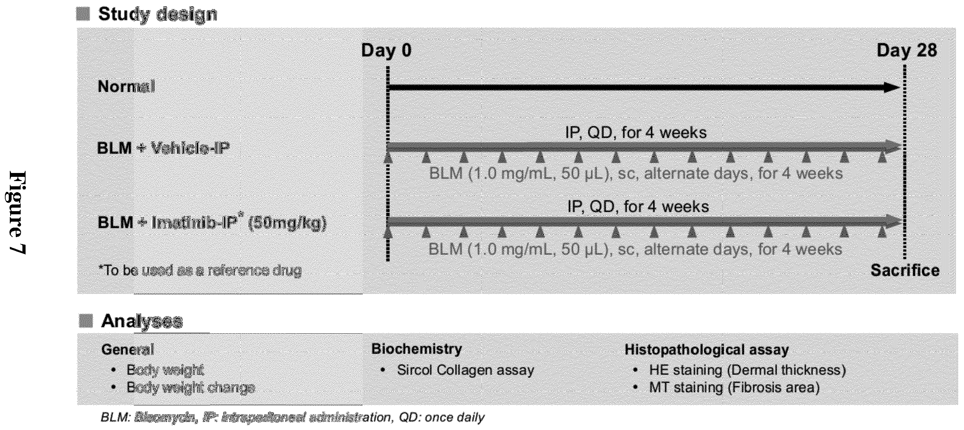

**Figure 7****. Study Design to Test New Treatments for Skin Fibrosis.** The study includes normal mice, bleomycin (BLM) + vehicle i.p., BLM + imatinib i.p. (50 mg/kg) as a positive control group and a BLM + anti-CLDNl-specific mAb. Clinical data, biochemistry and histopathological assays are performed and collected.

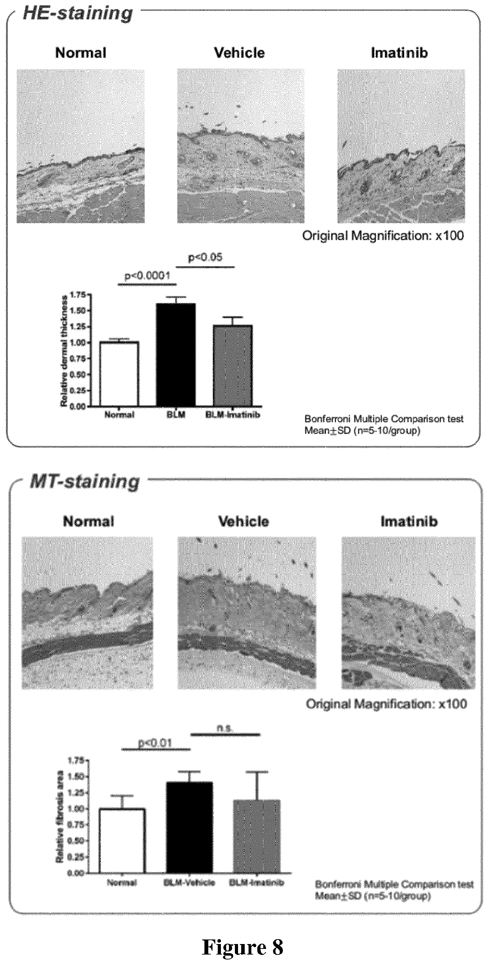

**Figure 8****. Dermal Thickness and Skin Fibrosis Quantification.** Hematoxylin and eosin (HE) and Masson Thricromic (MT) are used to quantified dermal thickness and skin fibrosis, respectively. Imatinib showed a significant effect in reducing dermal thickness and a trend in reduction of skin fibrosis.

### Definitions

Throughout the specification, several terms are employed that are defined in the following paragraphs.

As used herein, the term *"**subject**"* refers to a human or another mammal (e.g., primate, dog, cat, goat, horse, pig, mouse, rat, rabbit, and the like), that can develop a fibrotic disease, but may or may not be suffering from the disease. Non-human subjects may be transgenic or otherwise modified animals. In many embodiments of the present invention, the subject is a human being. In such embodiments, the subject is often referred to as an *"**individual**"* or a *"**patient**".* The term "individual" does not denote a particular age, and thus encompasses newborns, children, teenagers, and adults. The term *"patient"* more specifically refers to an individual suffering from a disease. In the practice of the present invention, a patient will generally be diagnosed with a fibrotic disease.

The term *"**treatment**"* is used herein to characterize a method or process that is aimed at (1) delaying or preventing the onset of a disease or condition (here a fibrotic disease); (2) slowing down or stopping the progression, aggravation, or deterioration of the symptoms of the disease or condition; (3) bringing about amelioration of the symptoms of the disease or condition; or (4) curing the disease or condition. A treatment may be administered prior to the onset of the disease or condition, for a prophylactic or preventive action. Alternatively or additionally, a treatment may be administered after initiation of the disease or condition, for a therapeutic action.

The terms *"**fibrotic disease**"* and *"**fibrotic disorder**"* are used herein interchangeably and have their art understood meaning. They refer to a clinical condition that is characterized by dysregulated tissue growth and scarring destroying healthy tissue, which can lead to disruption of normal function of virtually any organ of the body, including the heart, lung, kidney, liver and skin.

A *"**pharmaceutical composition**"* is defined herein as comprising an effective amount of at least one anti-Claudin-1 antibody (or a biologically active fragment thereof), and at least one pharmaceutically acceptable carrier or excipient.

As used herein, the term *"**effective amount**"* refers to any amount of a compound, agent, antibody, or composition that is sufficient to fulfil its intended purpose(s), *e.g.,* a desired biological or medicinal response in a cell, tissue, system or subject. For example, in certain embodiments of the present invention, the purpose(s) may be: to prevent the onset of a fibrotic disease, to slow down, alleviate or stop the progression, aggravation or deterioration of the symptoms of the fibrotic disease; to bring about amelioration of the symptoms of the disease, or to cure the disease.

The term *"**pharmaceutically acceptable carrier or excipient**"* refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, adsorption delaying agents, and the like. The use of such media and agents for pharmaceutically active substances is well known in the art (see for example *"*Remington's Pharmaceutical Sciences", E.W. Martin, 18th Ed., 1990, Mack Publishing Co.: Easton, PA, which is incorporated herein by reference in its entirety).

The term *"**human Claudin-1*** (or ***CLDN1***)" refers to a protein having the sequence shown in NCBI Accession Number NP_066924, or any naturally occurring variants commonly found in HCV permissive human populations. The term *"**extracellular domain**"* or *"**ectodomain**"* of Claudin-1 refers to the region of the Claudin-1 sequence that extends into the extracellular space (*i.e.,* the space outside a cell).

The term *"**antibody**",* as used herein, refers to any immunoglobulin that contains an antigen binding site that immunospecifically binds an antigen. As such, the term antibody encompasses not only whole antibody molecules, but also antibody fragments as well as variants (including derivatives) of antibodies and of antibody fragments as long as the derivatives and fragments maintain specific binding ability. The term encompasses monoclonal antibodies and polyclonal antibodies. The term also covers any protein having a binding domain, which is homologous or largely homologous to an immunoglobulin-binding domain. These proteins may be derived from natural sources, or partly or wholly synthetically produced.

The term *"**specific binding**",* when used in reference to an antibody, refers to an antibody binding to a predetermined antigen. Typically, the antibody binds with an affinity of at least 1 x 10⁷ M⁻¹, and binds to the predetermined antigen with an affinity that is at least two-fold greater than the affinity for binding to a non-specific antigen (*e.g.,* BSA, casein).

As used herein, the term *"**humanized antibody**"* refers to a chimeric antibody comprising amino acid residues from non-human hypervariable regions and amino acid residues from human framework regions (FRs). In particular, a humanized antibody comprises all or substantially all of at least one, typically two, variable domains, in which all or substantially all of the complementarity determining regions (CDRs) are those of a human antibody. A humanized antibody optionally may comprise at least a portion of an antibody constant region derived from a human antibody. A "humanized form" of an antibody, e.g., a non-human antibody, refers to an antibody that has undergone humanization.

The term *"**isolated**",* as used herein in reference to a protein or polypeptide, means a protein or polypeptide, which by virtue of its origin or manipulation is separated from at least some of the components with which it is naturally associated or with which it is associated when initially obtained. By "isolated", it is alternatively or additionally meant that the protein or polypeptide of interest is produced or synthesized by the hand of man.

The terms *"**protein**", "**polypeptide**",* and *"**peptide**"* are used herein interchangeably, and refer to amino acid sequences of a variety of lengths, either in their neutral (uncharged) forms or as salts, and either unmodified or modified by glycosylation, side-chain oxidation, or phosphorylation. In certain embodiments, the amino acid sequence is a full-length native protein. In other embodiments, the amino acid sequence is a smaller fragment of the full-length protein. In still other embodiments, the amino acid sequence is modified by additional substituents attached to the amino acid side chains, such as glycosyl units, lipids, or inorganic ions such as phosphates, as well as modifications relating to chemical conversions of the chains such as oxidation of sulfydryl groups. Thus, the term "protein" (or its equivalent terms) is intended to include the amino acid sequence of the full-length native protein, or a fragment thereof, subject to those modifications that do not significantly change its specific properties. In particular, the term "protein" encompasses protein isoforms, *i.e.,* variants that are encoded by the same gene, but that differ in their pI or MW, or both. Such isoforms can differ in their amino acid sequence (*e.g*., as a result of allelic variation, alternative splicing or limited proteolysis), or in the alternative, may arise from differential post-translational modification (*e.g*., glycosylation, acylation, phosphorylation).

The term *"**analog**",* as used herein in reference to a protein, refers to a polypeptide that possesses a similar or identical function as the protein but need not necessarily comprise an amino acid sequence that is similar or identical to the amino acid sequence of the protein or a structure that is similar or identical to that of the protein. Preferably, in the context of the present invention, a protein analog has an amino acid sequence that is at least 30%, more preferably, at least 35%, 40%, 45%, 50%, 55%, 60%, 65%, 70%, 75%, 80%, 85%, 90%, 95% or 99% identical to the amino acid sequence of the protein.

The term *"**fragment**"* and the term *"**portion**",* as used herein in reference to a protein, refers to a polypeptide comprising an amino acid sequence of at least 5 consecutive amino acid residues (preferably, at least about: 10, 15, 20, 25, 30, 35, 40, 50, 60, 70, 80, 90, 100, 125, 150, 175, 200, 250 or more consecutive amino acid residues) of the amino acid sequence of a protein. The fragment of a protein may or may not possess a functional activity of the protein.

The term *"**biologically active**",* as used herein to characterize a protein variant, analog or fragment, refers to a molecule that shares sufficient amino acid sequence identity or homology with the protein to exhibit similar or identical properties to the protein. For, example, in many embodiments of the present invention, a biologically active fragment of an anti-Claudin-1 antibody is a fragment that retains the ability of the whole antibody to bind to an antigen.

The term *"**homologous**"* (or *"**homology**"*), as used herein, is synonymous with the term "identity" and refers to the sequence similarity between two polypeptide molecules or between two nucleic acid molecules. When a position in both compared sequences is occupied by the same base or same amino acid residue, the respective molecules are then homologous at that position. The percentage of homology between two sequences corresponds to the number of matching or homologous positions shared by the two sequences divided by the number of positions compared and multiplied by 100. Generally, a comparison is made when two sequences are aligned to give maximum homology. Homologous amino acid sequences share identical or similar amino acid sequences. Similar residues are conservative substitutions for, or "allowed point mutations" of, corresponding amino acid residues in a reference sequence. "Conservative substitutions" of a residue in a reference sequence are substitutions that are physically or functionally similar to the corresponding reference residue, *e.g.* that have a similar size, shape, electric charge, chemical properties, including the ability to form covalent or hydrogen bonds, or the like. Particularly preferred conservative substitutions are those fulfilling the criteria defined for an "accepted point mutation" as described by Dayhoff et al. ("Atlas of Protein Sequence and Structure", 1978, Nat. Biomed. Res. Foundation, Washington, DC, Suppl. 3, 22: 354-352).

The terms *"**labeled**", "**labeled with a detectable agent**"* and *"**labeled with a detectable moiety**"* are used herein interchangeably. These terms are used to specify that an entity (*e.g.,* an antibody) can be visualized, for example, following binding to another entity (*e.g.,* an antigen). Preferably, a detectable agent or moiety is selected such that it generates a signal which can be measured and whose intensity is related (*e.g*., proportional) to the amount of bound entity. Methods for labeling proteins and polypeptides, including antibodies, are well-known in the art. Labeled polypeptides can be prepared by incorporation of, or conjugation to, a label, that is directly or indirectly detectable by spectroscopic, photochemical, biochemical, immunochemical, electrical, optical or chemical means, or any other suitable means. Suitable detectable agents include, but are not limited to, various ligands, radionuclides, fluorescent dyes, chemiluminescent agents, microparticles, enzymes, colorimetric labels, magnetic labels, and haptens.

The terms *"**approximately**"* and *"**about**"* as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention concerns the use of anti-Claudin-1 antibodies for the prevention and/or treatment of fibrotic diseases. In particular, the present invention relates to the use of anti-Claudin-1 antibodies for the prevention and/or treatment of pulmonary fibrosis, kidney fibrosis and skin fibrosis.

### I - Anti-Claudin-1 Antibodies

The present Inventors have previously developed monoclonal antibodies directed against human Claudin-1 and demonstrated that these monoclonal antibodies cure HCV infection *in vivo* without detectable adverse effects (see EP 08 305 597 and WO 2010/034812). They then showed that the anti-Claudin-1 monoclonal antibodies interfere with liver cell signalling and reverse a patient-derived hepatocellular carcinoma (HCC) risk signature in a liver cell-based model system, making them useful in the prevention and/or treatment of hepatocellular carcinoma (HCC) irrespective of the etiology (see EP 15 159 872 and WO 2016/146809). They have now demonstrated that anti-Claudin-1 antibodies can be used in the prevention or treatment of fibrotic diseases, in particular lung fibrosis, such as idiopathic pulmonary fibrosis (IPF).

Anti-Claudin-1 antibodies that can be used in the practice of the present invention include any antibody raised against Claudin 1. Examples of anti-Claudin-1 antibodies that can be used in the practice of the present invention include, in particular, the polyclonal and monoclonal anti-CLDN1 antibodies that were developed by the present Inventors (see EP 08 305 597 and WO 2010/034812, Fofana et al., Gastroenterology, 2010, 139(3): 953-64, 964.e1-4). As described in these documents, eight monoclonal antibodies were produced by genetic immunization and shown to efficiently inhibit HCV infection by targeting the extracellular domain of Claudin 1. The monoclonal anti-Claudin-1 antibodies are called OM-4A4-D4, OM-7C8-A8, OM-6D9-A6, OM-7D4-C1, OM-6E1-B5, OM-3E5-B6, OM-8A9-A3, and OM-7D3-B3. Thus, anti-Claudin-1 antibodies suitable for use in the practice of the present invention include monoclonal antibodies secreted by any one of the hybridoma cell lines deposited by INSERM (one of the present Applicants) and GENOVAC at the DSMZ (Deutsche Sammlung von Mikro-organismen und Zellkuturen GmbH, Inhoffenstraße 7 B, 38124 Braunschweig, Germany) on July 29, 2008 under Accession Numbers DSM ACC2931, DSM ACC2932, DSM ACC2933, DSM ACC2934, DSM ACC2935, DSM ACC2936, DSM ACC2937, and DSM ACC2938 (described in EP 08 305 597 and WO 2010/034812).

Other anti-Claudin-1 antibodies suitable for use in the practice of the present invention include monoclonal antibodies that have the same epitope as anti-Claudin-1 monoclonal antibodies secreted by any one of the hybridoma cell lines listed above. In certain embodiments, the epitope is strongly dependent on the conservation of the conserved motif W(30)-GLW(51)-C(54)-C(64) in Claudin-1 first extracellular loop (see EP 08 305 597 and WO 2010/034812).

Other examples of suitable anti-Claudin-1 antibodies include those disclosed in European Patent No. EP 1 167 389, in U.S. Patent No. 6,627,439, in international patent application published under No. WO 2014/132307, in international patent applications published under No. WO 2015/014659 and No. WO 2015/014357, and in Yamashita et al., J. Pharmacol. Exp. Ther., 2015, 353(1): 112-118.

Anti-Claudin-1 antibodies suitable for use in the present invention may be polyclonal antibodies or monoclonal antibodies.

Instead of using the hybridomas described above as a source of the antibodies, the anti-Claudin-1 antibodies may be prepared using any other suitable method known in the art. For example, an anti-Claudin-1 monoclonal antibody may be prepared by recombinant DNA methods. These methods generally involve isolation of the genes encoding the desired antibody, transfer of the genes into a suitable vector, and bulk expression in a cell culture system. The genes or DNA encoding the desired monoclonal antibody may be readily isolated and sequenced using conventional procedures (*e.g*., using oligonucleotide probes that are capable of binding specifically to genes encoding the heavy and light chains of murine antibodies). Hybridoma cell lines may serve as a preferred source of such DNA. Suitable host cells for recombinant production of antibodies include, but are not limited to, appropriate mammalian host cells, such as CHO, HeLa, or CV1. Suitable expression plasmids include, without limitation, pcDNA3.1 Zeo, pIND(SP1), pREP8 (all commercially available from Invitrogen, Carlsbad, CA, USA), and the like. The antibody genes may be expressed *via* viral or retroviral vectors, including MLV-based vectors, vaccinia virus-based vectors, and the like. Cells may be grown using standard methods, in suitable culture media such as, for example, DMEM and RPMI-1640 medium. The anti-Claudin-1 antibodies may be expressed as single chain antibodies. Isolation and purification of recombinantly produced antibodies may be performed by standard methods. For example, an anti-Claudin-1 monoclonal antibody may be recovered and purified from cell cultures by protein A purification, ammonium sulphate or ethanol precipitation, acid extraction, anion or cation exchange chromatography, phosphocellulose chromatography, hydrophobic interaction chromatography, affinity chromatography, such as Protein A column, hydroxylapatite chromatography, lectin chromatography, or any suitable combination of these methods. High performance liquid chromatography (HPLC) can also be employed for purification.

Alternatively, an anti-Claudin-1 antibody for use according to the present invention may be obtained from commercial sources.

In certain embodiments, an anti-Claudin-1 antibody is used in its native form. In other embodiments, it is truncated (*e.g., via* enzymatic cleavage or other suitable method) to provide immunoglobulin fragments or portions, in particular, fragments or portions that are biologically active. Biologically active fragments or portions of an anti-Claudin-1 antibody include fragments or portions that retain the ability of the antibody to bind to the antigen of the whole antibody, in particular to the extracellular domain of Claudin-1.

A biologically active fragment or portion of an anti-Claudin-1 antibody may be a Fab fragment or portion, a F(ab')₂ fragment or portion, a variable domain, or one or more CDRs (complementary determining regions) of the antibody (for example an antibody that comprises all 6 CDRs of an anti-Claudin-1 monoclonal antibody). Alternatively, a biologically active fragment or portion of an anti-Claudin-1 antibody may be derived from the carboxyl portion or terminus of the antibody protein and may comprise an Fc fragment, an Fd fragment or an Fv fragment.

Anti-Claudin-1 antibody fragments for use according to the present invention may be produced using any suitable method known in the art including, but not limited to, enzymatic cleavage (*e.g.*, proteolytic digestion of intact antibodies) or synthetic or recombinant techniques. For example, F(ab')₂, Fab, Fv and ScFv (single chain Fv) antibody fragments can be expressed in and secreted from mammalian host cells or from *E. coli.* Antibodies can also be produced in a variety of truncated forms using antibody genes in which one or more stop codons have been introduced upstream of the natural stop site. The various portions of antibodies can be joined together chemically by conventional techniques, or can be prepared as a contiguous protein using genetic engineering techniques.

Anti-Claudin-1 antibodies (or biologically active fragments thereof) suitable for use according to the present invention may be produced in a modified form, such as a fusion protein (*i.e.,* an immunoglobulin molecule or portion thereof linked to a polypeptide entity). Preferably, the fusion protein retains the biological activity of the antibody. A polypeptide entity to be fused to an anti-Claudin-1 antibody, or a biologically active fragment thereof, may be selected to confer any of a number of advantageous properties to the resulting fusion protein. For example, the polypeptide entity may be selected to provide increased expression of the recombinant fusion protein. Alternatively or additionally, the polypeptide entity may facilitate purification of the fusion protein, for example, by acting as a ligand in affinity purification. A proteolytic cleavage site may be added to the recombinant protein so that the desired sequence can ultimately be separated from the polypeptide entity after purification. The polypeptide entity may also be selected to confer an improved stability to the fusion protein, when stability is a goal. Examples of suitable polypeptide entities include, for example, polyhistidine tags, that allow for the easy purification of the resulting fusion protein on a nickel chelating column. Glutathione-S-transferase (GST), maltose B binding protein, or protein A are other examples of suitable polypeptide entities.

An anti-Claudin-1 antibody for use according to the present invention may be re-engineered so as to optimize stability, solubility, *in vivo* half-life, or ability to bind additional targets. Genetic engineering approaches as well as chemical modifications to accomplish any or all of these changes in properties are well known in the art. For example, the addition, removal, and/or modification of the constant regions of an antibody are known to play a particularly important role in the bioavailability, distribution, and half-life of therapeutically administered antibodies. The antibody class and subclass, determined by the Fc or constant region of the antibody (which mediates effector functions), when present, imparts important additional properties.

Additional fusion proteins of the invention may be generated through the techniques of DNA shuffling well known in the art (see, for example, U.S. Pat. Nos. 5,605,793; 5,811,238; 5,830,721; 5,834,252; and 5,837,458).

Anti-Claudin-1 antibodies suitable for use according to the present invention may also be "humanized": sequence differences between rodent antibodies and human sequences can be minimized by replacing residues which differ from those in the human sequences by site-directed mutagenesis of individual residues or by grafting of entire regions or by chemical synthesis. Humanized antibodies can also be produced using recombinant methods. In the humanized form of the antibody, some, most or all of the amino acids outside the CDR regions are replaced with amino acids from human immunoglobulin molecules, while some, most or all amino acids within one or more CDR regions are unchanged. Small additions, deletions, insertions, substitutions or modifications of amino acids are permissible as long as they do not significantly modify the biological activity of the resulting antibody. Suitable human "replacement" immunoglobulin molecules include IgG1, IgG2, IgG2a, IgG2b, IgG3, IgG4, IgA, IgM, IgD or IgE molecules, and fragments thereof. Alternatively, the T-cell epitopes present in rodent antibodies can be modified by mutation (de-immunization) to generate non-immunogenic rodent antibodies that can be applied for therapeutic purposes in humans (see webpage: accurobio.com).

In some embodiments, a humanized anti-Claudin-1 antibody for use according to the present invention is one previously described by the present Inventors in EP 16 305 317 and WO 2017/162678. Such a humanized anti-Claudin-1 monoclonal antibody comprises all of the CDRs of rat monoclonal antibody OM-7D3-B3 (secreted by hybridoma cell line deposited under Accession Number DSM ACC2938 - see above), wherein the variable heavy chain of OM-7D3-B3 consists of amino acid sequence SEQ ID NO: 1 and the variable light chain of OM-7D3-B3 consists of amino acid sequence SEQ ID NO: 2, said humanized antibody further comprising:
a) at least one antibody variable heavy chain (VH) consisting of the amino acid sequence of SEQ ID NO: 3 or SEQ ID NO: 4 or SEQ ID NO: 5, or
b) at least one antibody variable light chain (VL) consisting of the amino acid sequence of SEQ ID NO: 6 or SEQ ID NO: 7 or SEQ ID NO: 8,
   wherein SEQ ID NO: 1, the variable heavy chain of OM-7D3-B3 is the following amino acid sequence, wherein the CDRs are shown in bold and underlined: 
EVQLVESGGGLVQPGRSLKLSCLGSGFSFSSYGMNWIRQAPGKGLEWVA
SISPSGSYFYYADSVKGRFTISRENAKNTLYLQMTSLRAEDTALYYCARL
PGFNPPFDHWGQGVVVTVSS
   and wherein SEQ ID NO: 2, the variable light chain of OM-7D3-B3 is the following amino acid sequence, wherein the CDRs are shown in bold and underlined: 
NTVMTQSPTSMFMSVGDRVTMNCKASQNVGGNVDWYQWKPGQSPKL
LMYGASNRYTGVPDRFRGSGSGTDFTLTISNMQTEDLAVYYCLQYKNN
PWTFGGGTKVELK,
   wherein SEQ ID NO: 3 is the sequence of the humanized variable heavy chain H3: 
QVQLVESGGGVVQPGRSLRLSCLGSGFSFSSYGMNWVRQAPGKGLEWV
ASISPSGSYFYYADSVKGRFTISRDNSKNTLYLQMTSLRAEDTAIYYCARL
PGFNPPFDHWGQGTLVTVSS,
   wherein SEQ ID NO: 4 is the sequence of the humanized variable heavy chain H2: 
QVQLVESGGGVVQPGRSLRLSCAASGFSFSSYGMNWVRQAPGKGLEWV
TSISPSGSYFYYADSVKGRFTISRDNSKNTLYLQMNSLRAEDTAIYYCARL
PGFNPPFDHWGQGTLVTVSS,
   wherein SEQ ID NO: 5 is the sequence of the humanized variable heavy chain H1: 
EVQLVESGGGLVKPGGSLRLSCAASGFSFSSYGMNWVRQAPGKGLEWV
SSISPSGSYFYYADSVKGRFTISRDNAKNSLYLQMNSLRAEDTAVYYCAR
LPGFNPPFDHWGQGTLVTVSS,
   wherein SEQ ID NO: 6 is the sequence of the humanized variable light chain L3: 
DIQMTQSPSSLSASVGDRVTITCKASQNVGGNVDWYQWKPGKAPKLLIY
GASNRYTGVPDRFRGSGSGTDFTLTISSLQPEDVATYYCLQYKNNPWTFG
GGTKVEIK,
   wherein SEQ ID NO: 7 is the sequence of the humanized variable light chain L2: 
DIQMTQSPSSLSASVGDRVTITCKASQNVGGNVDWYQWKPGKAPKLLIY
GASNRYTGVPSRFRGSGSGTDFTLTISSLQPEDVATYYCLQYKNNPWTFG
QGTKVEIK, and
   wherein SEQ ID NO: 8 is the sequence of the humanized variable light chain L1:

DIQMTQSPATLSVSPGERATLSCKASQNVGGNVDWYQWKPGQAPRLLIY
GASNRYTGIPARFRGSGSGTEFTLTISSLQSEDFAVYYCLQYKNNPWTFG
QGTKVEIK.

For example, such a humanized anti-Claudin-1 monoclonal antibody may comprise:
a) two antibody variable heavy chains (VH), both variable heavy chains consisting of the amino acid sequence of SEQ ID NO: 3 or SEQ ID NO: 4 or SEQ ID NO: 5, or
b) two antibody variable light chains (VL), both variable light chains consisting of the amino acid sequence of SEQ ID NO: 6 or SEQ ID NO: 7 or SEQ ID NO: 8.

For example, such a humanized anti-Claudin-1 monoclonal antibody may comprise;
1) two antibody variable heavy chains (VH) consisting of the amino acid sequence SEQ ID NO: 3, and two antibody variable light chains (VL) consisting of the amino acid sequence SEQ ID NO: 6 [*H3L3*]; or
2) two antibody variable heavy chains (VH) consisting of the amino acid sequence SEQ ID NO: 3, and two antibody variable light chains (VL) consisting of the amino acid sequence SEQ ID NO: 7 [*H3L1*]*;* or
3) two antibody variable heavy chains (VH) consisting of the amino acid sequence SEQ ID NO: 3, and two antibody variable light chains (VL) consisting of the amino acid sequence SEQ ID NO: 8 [*H3L2*]; or
4) two antibody variable heavy chains (VH) consisting of the amino acid sequence SEQ ID NO: 4, and two antibody variable light chains (VL) consisting of the amino acid sequence SEQ ID NO: 6 [*H1L3*]*;* or
5) two antibody variable heavy chains (VH) consisting of the amino acid sequence SEQ ID NO: 4, and two antibody variable light chains (VL) consisting of the amino acid sequence SEQ ID NO: 7 [*H1L1*]*;* or
6) two antibody variable heavy chains (VH) consisting of the amino acid sequence SEQ ID NO: 4, and two antibody variable light chains (VL) consisting of the amino acid sequence SEQ ID NO: 8 [*H1L2*]*;* or
7) two antibody variable heavy chains (VH) consisting of the amino acid sequence SEQ ID NO: 5, and two antibody variable light chains (VL) consisting of the amino acid sequence SEQ ID NO: 6 [*H2L3*]; or
8) two antibody variable heavy chains (VH) consisting of the amino acid sequence SEQ ID NO: 5, and two antibody variable light chains (VL) consisting of the amino acid sequence SEQ ID NO: 7 [*H2L1*]*;* or
9) two antibody variable heavy chains (VH) consisting of the amino acid sequence SEQ ID NO: 5, and two antibody variable light chains (VL) consisting of the amino acid sequence SEQ ID NO: 8 [*H2L2*].

The humanized anti-Claudin-1 antibody may be a full monoclonal antibody having an isotope selected from the group consisting of IgG1, IgG2, IgG3 and IgG4. Alternatively, the humanized anti-Claudin-1 antibody may be a fragment of a monoclonal antibody selected from the group consisting of Fv, Fab, F(ab')2, Fab', dsFv, scFv, sc(Fv)2 and diabodies.

Anti-Claudin-1 antibodies (or biologically active variants or fragments thereof) suitable for use according to the present invention may be functionally linked (*e.g.,* by chemical coupling, genetic fusion, non-covalent association or otherwise) to one or more other molecular entities. Methods for the preparation of such modified antibodies (or conjugated antibodies) are known in the art (see, for example, *"*Affinity Techniques. Enzyme Purification: Part B", Methods in Enzymol., 1974, Vol. 34, Jakoby and Wilneck (Eds.), Academic Press: New York, NY; and Wilchek and Bayer, Anal. Biochem., 1988, 171: 1-32). Preferably, molecular entities are attached at positions on the antibody molecule that do not interfere with the binding properties of the resulting conjugate, *e.g.,* positions that do not participate in the specific binding of the antibody to its target.

The antibody molecule and molecular entity may be covalently, directly linked to each other. Or, alternatively, the antibody molecule and molecular entity may be covalently linked to each other through a linker group. This can be accomplished by using any of a wide variety of stable bifunctional agents well known in the art, including homofunctional and heterofunctional linkers.

In certain embodiments, an anti-Claudin-1 antibody (or a biologically active fragment thereof) for use according to the present invention is conjugated to a detectable agent. Any of a wide variety of detectable agents can be used, including, without limitation, various ligands, radionuclides (*e.g*., ³H, ¹²⁵I, ¹³¹I, and the like), fluorescent dyes (*e.g.*, fluorescein isothiocyanate, rhodamine, phycoerytherin, phycocyanin, allophycocyanin, o-phthalaldehyde and fluorescamine), chemiluminescent agents (*e.g.*, luciferin, luciferase and aequorin), microparticles (such as, for example, quantum dots, nanocrystals, phosphors and the like), enzymes (such as, for example, those used in an ELISA, *i.e.,* horseradish peroxidase, beta-galactosidase, luciferase, alkaline phosphatase), colorimetric labels, magnetic labels, and biotin, dioxigenin or other haptens and proteins for which antisera or monoclonal antibodies are available.

Other molecular entities that can be conjugated to an anti-Claudin-1 antibody of the present invention (or a biologically active fragment thereof) include, but are not limited to, linear or branched hydrophilic polymeric groups, fatty acid groups, or fatty ester groups.

Thus, in the practice of the present invention, anti-Claudin-1 antibodies can be used under the form of full length antibodies, biologically active variants or fragments thereof, chimeric antibodies, humanized antibodies, and antibody-derived molecules comprising at least one complementary determining region (CDR) from either a heavy chain or light chain variable region of an anti-Claudin-1 antibody, including molecules such as Fab fragments, F(ab')₂ fragments, Fd fragments, Fabc fragments, Sc antibodies (single chain antibodies), diabodies, individual antibody light single chains, individual antibody heavy chains, chimeric fusions between antibody chains and other molecules, and antibody conjugates, such as antibodies conjugated to a therapeutic agent or a detectable agent. Preferably, anti-Claudin-1 antibody-related molecules according to the present invention retain the antibody's ability to bind its antigen, in particular the extracellular domain of Claudin-1.

One skilled in the art will understand that other compounds targeting Claudin-1, can be used in the practice of the present invention, including, but not limited to, small molecules and siRNAs.

### II - Treatment and/or Prevention of a Fibrotic Disease

### A. Indications

The present Inventors have shown that anti-Claudin-1 antibodies specifically accumulate in the lungs, the kidneys, and the skin, and are able to prevent and treat lung fibrosis in a state-of-the-art model of idiopathic pulmonary fibrosis (IPF) without any significant adverse effects. Therefore, anti-Claudin-1 antibodies, or biologically active fragments thereof, as defined above, may be used in methods to prevent and/or treat pulmonary fibrosis, kidney fibrosis, and skin fibrosis.

Methods of treatment of the present invention may be accomplished using an anti-Claudin-1 antibody, or a biologically active fragment thereof, or a pharmaceutical composition comprising such an antibody or fragment (see below). These methods generally comprise administration of an effective amount of an anti-Claudin-1 antibody, or biologically active fragment thereof, or of a pharmaceutical composition thereof, to a subject in need thereof (*i.e.,* a patient diagnosed with lung fibrosis or with kidney fibrosis or with skin fibrosis). Administration may be performed using any of the administration methods known to one skilled in the art (see below).

***Pulmonary Fibrosis.*** The terms *"**lung fibrosis**"* and *"**pulmonary fibrosis**"* are used herein interchangeably. They refer to a number of conditions, of known or unknown etiologies, that cause interstitial lung damage, followed by fibrosis and eventually loss of lung elasticity. These conditions lead to symptoms such as persistent cough, chest pain, difficulty breathing and fatigue.

Pulmonary fibrosis that can be treated according to a method of the present invention may be caused by any of a variety of factors including, but not limited to, long-term exposure to certain toxins (*e.g.*, silica dust, asbestos fibers, hard metal dusts, coal dusts, grain dusts, bird and animal droppings); certain medical conditions (*e.g.*, dermatomyositis, polymyositis, mixed connective tissue disease, systemic lupus erythematosus, rheumatoid arthritis, sarcoidosis, scleroderma and pneumonia); radiation therapy (*e.g.,* for lung or breast cancer); and some medications (*e.g.*, chemotherapy drugs such as methotrexate and cyclophosphamide, heart medication such as amiodarone; some antibiotics such as nitrofurantoin and ethambutol; and anti-inflammatory drugs such as rituximab and sulfasalazine).

In some embodiments, pulmonary fibrosis that can be treated according to a method of the present invention may have no clear underlying cause. The term idiopathic pulmonary fibrosis is then used.

In some embodiments, the pulmonary fibrosis to be treated using a method of treatment of the present invention is selected from the group consisting of idiopathic pulmonary fibrosis (IPF), idiopatic nonspecific interstitial pneumonitis (NSIP), cryptogenic organizing pneumonia (COP), Hamman-Rich syndrome (also known as acute interstitial pneumonia), lymphocytic interstitial pneumonitis (LIP), respiratory bronchiolitis interstitial lung disease, desquamative interstitial pneumonitis or idiopathic lymphoid interstitial pneumonia, and idiopathic pleuroparenchymal fibroelastosis.

In certain preferred embodiments, the pulmonary fibrosis is idiopathic pulmonary fibrosis.

In some embodiments, the pulmonary fibrosis is associated with chronic obstructive pulmonary disease. Chronic obstructive pulmonary disease (COPD) is a type of progressive respiratory disease characterized by airway obstruction, long-term breathing problems and poor airflow.

Administration of an anti-Claudin-1 antibody, or of a pharmaceutical composition thereof, to patients suffering from pulmonary fibrosis may slow, reduce, stop or alleviate the progression of the disease, in particular the development of complications such as pulmonary hypertension, right-sided heart failure, respiratory failure, lung cancer, or other lung complications such as blood clots in the lung, a collapsed lung or lung infections.

Alternatively or additionally, administration of an anti-Claudin-1 antibody, or of a pharmaceutical composition thereof, to a patient suffering from pulmonary fibrosis may result in amelioration of at least one of the symptoms experienced by the individual including, but not limited to, dry cough, shortness of breath, fatigue, muscle pain, join pain, and weight loss.

Alternatively or additionally, administration of an anti-Claudin-1 antibody, or of a pharmaceutical composition thereof, to a patient suffering from pulmonary fibrosis may help avoiding or, at least delaying, lung transplantation.

In some embodiments, a method of the invention is administered to a subject with a risk of developing pulmonary fibrosis, for example someone who has been exposed to certain toxins known to be associated with lung fibrosis or someone who has received radiation therapy, or yet someone who has been treated with certain medications. Administration of an anti-Claudin-1 antibody, or of a pharmaceutical composition thereof, may result in the prevention of the development of the disease or in the prevention of the progression of the disease beyond the very early stages.

The effects of a treatment according to the invention may be monitored using any of the assays and tests known in the art for the diagnosis of pulmonary fibrosis affecting the patient. Such assays and tests include, but are not limited to, imaging tests (such as chest X-ray, computerized tomography (CT) scan, and echocardiogram); lung function tests (such as pulmonary functions testing (*e.g.*, spirometry), pulse oximetry, exercise stress test, and arterial blood gas test); or biopsy by bronchoscopy or surgical biopsy.

In certain embodiments of a method of prevention or treatment of pulmonary fibrosis according to the present invention, an anti-Claudin-1 antibody (or a biologically active fragment thereof), or a pharmaceutical composition thereof, is administered alone. In other embodiments, an anti-Claudin-1 antibody (or a biologically active fragment thereof), or a pharmaceutical composition thereof, is administered in combination with at least one additional therapeutic agent and/or therapeutic procedure. The anti-Claudin-1 antibody (or biologically active fragment thereof), or pharmaceutical composition thereof, may be administered prior to administration of the therapeutic agent or therapeutic procedure, concurrently with the therapeutic agent or therapeutic procedure, and/or following administration of the therapeutic agent or therapeutic procedure.

Therapeutic agents that may be administered in combination with an anti-Claudin-1 antibody (or biologically active fragment thereof), or a pharmaceutical composition thereof, may be selected among a large variety of biologically active compounds that are known in the art to have a beneficial effect in the treatment or management of pulmonary fibrosis. Examples of such therapeutic agents include, but are not limited to, immunosuppressive agents such as corticosteroids, anti-fibrotic agents such as ciclosporin or colchicine, new medications such as pirfenidone (ESBRIET®) and nintedanib (OFEV®), which have been approved by the Food and Drug Administration (FDA); and anti-acid medications to treat gastroesophageal reflux disease (GERD), a digestive condition that commonly occurs in people with idiopathic pulmonary fibrosis. Examples of therapeutic procedures include, but are not limited to oxygen therapy, which makes breathing and exercise easier, prevents or lessens complications from low blood oxygen levels, reduces blood pressure in the right side of the heart and improves sleep and sense of well-being; pulmonary rehabilitation, which helps manage the symptoms and improves daily functioning by improving physical endurance and lung efficiency; and lung transplant, which improves the quality of life and allows patients to live a longer life.

Thus, in certain embodiments, the method of treatment of pulmonary fibrosis according to the invention is administered in combination with a therapeutic agent selected from the group consisting of corticosteroids, ciclosporin, colchicine, pirfenidone, nintedanib and anti-acid drugs to treat gastroesophageal reflux disease (GERD). Alternatively or additionally, the method of treatment of pulmonary fibrosis according to the invention is administered in combination with a therapeutic procedure selected from the group consisting of lung transplantation, hyperbaric oxygen therapy and pulmonary rehabilitation.

***Kidney Fibrosis**.* The terms *"**kidney fibrosis**"* and *"**renal fibrosis**"* are used herein interchangeably. Renal fibrosis is the hallmark of chronic kidney disease, regardless of underlying etiology. The pathological finding of renal fibrosis is characterized by progressive tissue scarring including glomerulosclerosis, tubulointerstitial fibrosis and loss of renal parenchyma (including tubular atrophy, loss of capillaries and podocytes). All the renal diseases are accompanied by kidney fibrosis, which is a progressive process that ultimately leads to end-stage renal failure (ESRD), a devastating disorder that requires dialysis or kidney transplant. Since chronic deterioration of renal function depends heavily on the extent of fibrosis of the kidney, it is thought that inhibiting the progress of fibrosis can result in suppression of the development of chronic renal failure. The term "chronic renal failure" refers to a state in which the renal functions gradually deteriorate irreversibly and homeostasis of a living body cannot be maintained.

Renal fibrosis that can be treated according to a method of the present invention may be caused by any of a variety of factors including, but not limited to, certain medical conditions (nephropathies such as glomerular diseases (e.g., glomerulosclerosis, glomerulonephritis), chronic renal insufficiency, acute kidney injury, high blood pressure, polycystic kidney disease, vesicoureteral reflux, pyelonephritis (recurrent kidney infection), and autoimmune diseases such as diabetes mellitus); certain medical interventions (such as nephrectomy or kidney removal, a procedure which is sometimes performed on patients with kidney cancer and which may negatively impact kidney function of the remaining kidney; dialysis following kidney failure; and catheter placement); and some medications (chemotherapy and immunosuppressive therapy, that are a source of harmful effects to the kidney which result in most of the cases in renal fibrosis; long-time use of lithium and of non-steroidal anti-inflammatory drugs).

In some embodiments, renal fibrosis to be treated using a method of treatment of the present invention is selected from the group consisting of renal interstitial fibrosis and glomerulosclerosis.

Administration of an anti-Claudin-1 antibody, or of a pharmaceutical composition thereof, to patients suffering from kidney fibrosis may slow, reduce, stop or alleviate the progression of the disease, in particular the development of complications such as fluid retention including pulmonary edema; hyperkalemia (sudden rise of potassium levels in the blood); cardiovascular disease; decreased immune response; pericarditis; and end-stage kidney disease.

Alternatively or additionally, administration of an anti-Claudin-1 antibody, or of a pharmaceutical composition thereof, to a patient suffering from renal fibrosis may result in amelioration of at least one of the symptoms experienced by the individual including, but not limited to, nausea, vomiting, loss of appetite, fatigue and weakness, muscle cramps, fluid retention (puffiness or swelling), chest pain, shortness of breath, and hypertension.

Alternatively or additionally, administration of an anti-Claudin-1 antibody, or of a pharmaceutical composition thereof, to a patient suffering from renal fibrosis may help avoiding, or at least delaying, dialysis or kidney transplant.

The effects of a treatment according to the invention may be monitored using any of the assays and tests known in the art for the diagnosis of renal fibrosis affecting the patient. Such assays and tests include, but are not limited to, imaging tests (such as ultrasound); blood tests (determination of creatinine and urea levels); urine tests, and biopsy.

In certain embodiments, an anti-Claudin-1 antibody (or a biologically active fragment thereof), or a pharmaceutical composition thereof, is administered alone. In other embodiments, an anti-Claudin-1 antibody (or a biologically active fragment thereof), or a pharmaceutical composition thereof, is administered in combination with at least one additional therapeutic agent and/or therapeutic procedure. The anti-Claudin-1 antibody (or biologically active fragment thereof), or pharmaceutical composition thereof, may be administered prior to administration of the therapeutic agent or therapeutic procedure, concurrently with the therapeutic agent or therapeutic procedure, and/or following administration of the therapeutic agent or therapeutic procedure.

Therapeutic agents that may be administered in combination with an anti-Claudin-1 antibody (or biologically active fragment thereof), or a pharmaceutical composition thereof, may be selected among a large variety of biologically active compounds that are known in the art to have a beneficial effect in the treatment or management of renal fibrosis. Examples of such therapeutic agents include, but are not limited to, anti-hypertensive drugs (in order to alleviate the burden on the glomerulus); supplementation in 1,25-dihydroxyvitamin D3 or erythropoietin, which are secreted by the kidney; angiotensin converting enzyme inhibitors (such as captoril, enalapril, delapril, imidapril, quinapril, temocapril, perindopril erbumine, and lisinopril) and angiotensin II receptor antagonists (such as losartan, valsartan, candesartan cilexetil, telmisartan, olmesartan medoxomil, and irbesartan), which are known to have a renal protective effect *per se* in addition to suppressing the progress of renal failure by decreasing glomerular blood pressure; diuretics, which relieve swelling; AST-120 (KREMEZIN®), an adsorptive carbon that adsorbs harmful substance in the intestine; and calcium polystyrene sulfonate, an ion-exchange resin that adorbs potassium in the intestine. Examples of therapeutic procedures include dialysis and kidney transplantation. The dialysis may be a hemodialysis or a peritoneal dialysis. In hemodialysis, a machine filters waste and excess fluids from the blood. In peritoneal dialysis, a catheter inserted in the abdomen fills the abdominal cavity with a dialysis solution that absorbs waste and excess fluids. After a period of time, the dialysis solution drains from the body, carrying the waste with it.

Thus, in certain embodiments, the method of treatment of kidney fibrosis according to the invention is administered in combination with a therapeutic agent selected from the group consisting of anti-hypertensive drugs 1,25-dihydroxyvitamin D3, erythropoietin, angiotensin converting enzyme inhibitors, angiotensin II receptor antagonists AST-120 (KREMEZIN®), and calcium polystyrene sulfonate. Alternatively or additionally, the method of treatment of kidney fibrosis according to the invention is administered in combination with a therapeutic procedure selected from the group consisting of dialysis and kidney transplantation.

***Skin Fibrosis**.* The terms *"**skin fibrosis**", "**dermal fibrosis**"* and *"**cutaneous fibrosis**"* are used herein interchangeably. They refer to an excessive scarring of the skin which results from a pathologic wound healing response. Skin fibrosis is characterized by fibroblast proliferation and excessive synthesis as well as deposition of extracellular matrix (ECM) proteins, such as collagen, elastin, and fibrillin. Clinically, skin fibrosis manifests as thickened, tightened and hardened areas of skin. Ultimately, skin fibrosis may lead to dermal contractures that affect the ability to flex and extend the joints. Despite the morbidity and socioeconomic burdens associated with skin fibrosis, there are limited effective therapeutic options. Current therapies are associated with significant side effects and even with combination therapy, progression, and recurrence often occurs.

Skin fibrosis that can be treated according to a method of the present invention may be caused by any of a variety of factors including, but not limited to, certain medical conditions (scleroderma in both localized (morphea, linear scleroderma) and systemic forms, graft-versus-host disease (GVHD), nephrogenic fibrosing dermopathy, mixed connective tissue disease, scleredema, scleromyxedema, eosinophilic fasciitis, chromoblastomycosis, hypertrophic scars and keloids); certain medical interventions (radiotherapy-induced skin fibroses); environmental or professional exposures to various chemicals (*e.g.*, in eosinophilia-myalgia syndrome induced by L-tryptophan); and exposure to certain physical agents (*e.g*., skin fibroses induced by physical trauma, surgical injury, heat or ice skin burns).

Administration of an anti-Claudin-1 antibody, or of a pharmaceutical composition thereof, to patients suffering from skin fibrosis may slow, reduce, stop or alleviate the progression of the skin disease, for example the propagation of fibrosis to a non-affected skin area, and/or may slow, reduce, stop or alleviate the development of complications such as disfigurement, dermal contractures, diminished function of an affected limb and propagation to internal organs.

Alternatively or additionally, administration of an anti-Claudin-1 antibody, or of a pharmaceutical composition thereof, to a patient suffering from skin fibrosis may result in amelioration of at least one of the symptoms experienced by the individual including, but not limited to, thickened, tightened and hardened areas of skin.

The effects of a treatment according to the invention may be monitored using any of the assays and tests known in the art for the diagnosis of dermal fibrosis affecting the patient. Such assays and tests make use of, for example, durometers for measuring skin hardness and/or tautness, cutometers for quantifying skin elasticity, ultrasonographic devices for assessing local dermal and subcutaneous blood flow, and digital infrared thermal imaging of skin. Other non-invasive methods of skin fibrosis diagnosis include ultrasound scan, elastography, confocal microscopy, and optical coherence tomography.

In certain embodiments of a method of prevention or treatment of dermal fibrosis according to the present invention, an anti-Claudin-1 antibody (or a biologically active fragment thereof), or a pharmaceutical composition thereof, is administered alone. In other embodiments, an anti-Claudin-1 antibody (or a biologically active fragment thereof), or a pharmaceutical composition thereof, is administered in combination with at least one additional therapeutic agent and/or therapeutic procedure. The anti-Claudin-1 antibody (or biologically active fragment thereof), or pharmaceutical composition thereof, may be administered prior to administration of the therapeutic agent and/or the therapeutic procedure, concurrently with the therapeutic agent and/or the therapeutic procedure, and/or following administration of the therapeutic agent and/or the therapeutic procedure.

Therapeutic agents that may be administered in combination with an anti-Claudin-1 antibody (or biologically active fragment thereof), or a pharmaceutical composition thereof, may be selected among immunosuppressive drugs (such as methotrexate, mycophenolyate, mofetil, cyclophosphamide and cyclosporine), tocilizumab (an anti-IL-6 receptor antibody), rituximab (an anti-CD20 antibody), and fresolimumab (an anti-TGF-β antibody, which show promising clinical outcomes.

Alternatively or additionally, the anti-Claudin-1 antibody (or biologically active fragment thereof), or a pharmaceutical composition thereof, may be administered in combination with a therapeutic procedure used in the treatment of skin fibrosis, such as ultraviolet phototherapy.

### B. Administration

An anti-Claudin-1 antibody, or a biologically active fragment thereof, (optionally after formulation with one or more appropriate pharmaceutically acceptable carriers or excipients), in a desired dosage, can be administered to a subject in need thereof by any suitable route. Various delivery systems are known and can be used to administer antibodies, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, and oral routes. An anti-Claudin-1 antibody, or a biologically active fragment thereof, or a pharmaceutical composition thereof, may be administered by any convenient or other appropriate route, for example, by infusion or bolus injection, by absorption through epithelial or mucosa linings (e.g., oral mucosa, bronchial mucosa, rectal and intestinal mucosa, etc). Administration can be systemic or local. As will be appreciated by those of ordinary skill in the art, in embodiments where an inventive antibody is administered in combination with an additional therapeutic agent, the antibody and therapeutic agent may be administered by the same route (*e.g.,* intravenously) or by different routes (*e.g.,* intravenously and orally).

### C. Dosage

An anti-Claudin-1 antibody, or a biologically active fragment thereof, (optionally after formulation with one or more appropriate pharmaceutically acceptable carriers or excipients), will be administered in a dosage such that the amount delivered is effective for the intended purpose. The route of administration, formulation and dosage administered will depend upon the therapeutic effect desired, the severity of the condition to be treated if already present, the presence of any infection, the age, sex, weight, and general health condition of the patient as well as upon the potency, bioavailability, and *in vivo* half-life of the antibody or composition used, the use (or not) of concomitant therapies, and other clinical factors. These factors are readily determinable by the attending physician in the course of the therapy. Alternatively or additionally, the dosage to be administered can be determined from studies using animal models (*e.g.*, chimpanzee or mice). Adjusting the dose to achieve maximal efficacy based on these or other methods are well known in the art and are within the capabilities of trained physicians. As studies are conducted using anti-Claudin-1 antibodies, further information will emerge regarding the appropriate dosage levels and duration of treatment.

A treatment according to the present invention may consist of a single dose or multiple doses. Thus, administration of an anti-Claudin-1 antibody, or a biologically active fragment thereof, (or a pharmaceutical composition thereof) may be constant for a certain period of time or periodic and at specific intervals, *e.g.*, hourly, daily, weekly (or at some other multiple day interval), monthly, yearly (*e.g.,* in a time release form). Alternatively, the delivery may occur at multiple times during a given time period, e.g., two or more times per week; two or more times per month, and the like. The delivery may be continuous delivery for a period of time, e.g., intravenous delivery.

In general, the amount of anti-Claudin-1 antibody, or a biologically active fragment thereof, (or a pharmaceutical composition thereof) administered will preferably be in the range of about 1 ng/kg to about 100 mg/kg body weight of the subject, for example, between about 100 ng/kg and about 50 mg/kg body weight of the subject; or between about 1 µg/kg and about 10 mg/kg body weight of the subject, or between about 100 µg/kg and about 1 mg/kg body weight of the subject.

### III - Pharmaceutical Compositions

As mentioned above, anti-Claudin-1 antibodies (and related molecules) may be administered *per se* or as a pharmaceutical composition. Accordingly, the present invention provides pharmaceutical compositions comprising an effective amount of an anti-Claudin-1 antibody, or a biologically active fragment thereof, described herein and at least one pharmaceutically acceptable carrier or excipient for use in the prevention and/or treatment of fibrotic diseases, in particular pulmonary fibrosis, kidney fibrosis and skin fibrosis. In some embodiments, the composition further comprises one or more additional biologically active agents.

The antibodies or pharmaceutical compositions may be administered in any amount and using any route of administration effective for achieving the desired prophylactic and/or therapeutic effect. The optimal pharmaceutical formulation can be varied depending upon the route of administration and desired dosage. Such formulations may influence the physical state, stability, rate of *in vivo* release, and rate of *in vivo* clearance of the administered active ingredient.

The pharmaceutical compositions of the present invention may be formulated in dosage unit form for ease of administration and uniformity of dosage. The expression "unit dosage form", as used herein, refers to a physically discrete unit of an anti-Claudin-1 antibody, or a biologically active fragment thereof, for the patient to be treated. It will be understood, however, that the total daily dosage of the compositions will be decided by the attending physician within the scope of sound medical judgement.

### A. Formulation

Injectable preparations, for example, sterile injectable aqueous or oleaginous suspensions may be formulated according to the known art using suitable dispersing or wetting agents, and suspending agents. The sterile injectable preparation may also be a sterile injectable solution, suspension or emulsion in a non-toxic parenterally acceptable diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspending medium. For this purpose, any bland fixed oil can be employed including synthetic mono- or di-glycerides. Fatty acids such as oleic acid may also be used in the preparation of injectable formulations. Sterile liquid carriers are useful in sterile liquid form compositions for parenteral administration.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Liquid pharmaceutical compositions which are sterile solutions or suspensions can be administered by, for example, intravenous, intramuscular, intraperitoneal or subcutaneous injection. Injection may be *via* single push or by gradual infusion. Where necessary or desired, the composition may include a local anesthetic to ease pain at the site of injection.

In order to prolong the effect of an active ingredient (here an anti-Claudin-1 antibody, or a biologically active fragment thereof), it is often desirable to slow the absorption of the ingredient from subcutaneous or intramuscular injection. Delaying absorption of a parenterally administered active ingredient may be accomplished by dissolving or suspending the ingredient in an oil vehicle. Injectable depot forms are made by forming micro-encapsulated matrices of the active ingredient in biodegradable polymers such as polylactide-polyglycolide. Depending upon the ratio of active ingredient to polymer and the nature of the particular polymer employed, the rate of ingredient release can be controlled. Examples of other biodegradable polymers include poly(orthoesters) and poly(anhydrides). Depot injectable formulations can also be prepared by entrapping the active ingredient in liposomes or microemulsions which are compatible with body tissues.

Liquid dosage forms for oral administration include, but are not limited to, pharmaceutically acceptable emulsions, microemulsions, solutions, suspensions, syrups, elixirs, and pressurized compositions. In addition to the anti-Claudin-1 antibody, or biologically active fragment thereof, the liquid dosage form may contain inert diluents commonly used in the art such as, for example, water or other solvents, solubilising agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cotton seed, ground nut, corn, germ, olive, castor, and sesame oils), glycerol, tetrahydrofurfuryl alcohol, polyethylene glycols, and fatty acid esters of sorbitan and mixtures thereof. Besides inert diluents, the oral compositions can also include adjuvants such as wetting agents, suspending agents, preservatives, sweetening, flavouring, and perfuming agents, thickening agents, colors, viscosity regulators, stabilizes or osmo-regulators. Examples of suitable liquid carriers for oral administration include water (potentially containing additives as above, e.g., cellulose derivatives, such as sodium carboxymethyl cellulose solution), alcohols (including monohydric alcohols and polyhydric alcohols such as glycols) and their derivatives, and oils (e.g., fractionated coconut oil and arachis oil). For pressurized compositions, the liquid carrier can be halogenated hydrocarbon or other pharmaceutically acceptable propellant.

Solid dosage forms for oral administration include, for example, capsules, tablets, pills, powders, and granules. In such solid dosage forms, an anti-Claudin-1 antibody, or a biologically active fragment thereof, may be mixed with at least one inert, physiologically acceptable excipient or carrier such as sodium citrate or dicalcium phosphate and one or more of: (a) fillers or extenders such as starches, lactose, sucrose, glucose, mannital, and silicic acid; (b) binders such as, for example, carboxymethylcellulose, alginates, gelatine, polyvinylpyrrolidone, sucrose, and acacia; (c) humectants such as glycerol; (d) disintegrating agents such as agar-agar, calcium carbonate, potato or tapioca starch, alginic acid, certain silicates, and sodium carbonate; (e) solution retarding agents such as paraffin; absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulphate, and mixtures thereof. Other excipients suitable for solid formulations include surface modifying agents such as non-ionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. In the case of capsules, tablets and pills, the dosage form may also comprise buffering agents.

Solid compositions of a similar type may also be employed as fillers in soft and hard-filled gelatine capsules using such excipients as lactose or milk sugar as well as high molecular weight polyethylene glycols and the like. The solid dosage forms of tablets, dragees, capsules, pills, and granules can be prepared with coatings and shells such as enteric coatings, release controlling coatings and other coatings well known in the pharmaceutical formulating art. They may optionally contain opacifying agents and can also be of a composition such that they release the active ingredient(s) only, or preferably, in a certain part of the intestinal tract, optionally, in a delaying manner. Examples of embedding compositions which can be used include polymeric substances and waxes.

In certain embodiments, it may be desirable to administer an inventive composition locally to an area in need of treatment (*e.g.*, the lung, the kidney or the skin). This may be achieved, for example, and not by way of limitation, by local infusion during surgery (*e.g.*, transplantation), topical application, by injection, by means of a catheter, by means of a stent or other implant or yet by means of an inhaler.

For topical administration, the composition may preferably be formulated as a gel, an ointment, a lotion, or a cream which can include carriers such as water, glycerol, alcohol, propylene glycol, fatty alcohols, triglycerides, fatty acid esters, or mineral oil. Other topical carriers include liquid petroleum, isopropyl palmitate, polyethylene glycol, ethanol (95%), polyoxyethylenemonolaurat (5%) in water, or sodium lauryl sulphate (5%) in water. Other materials such as antioxidants, humectants, viscosity stabilizers, and similar agents may be added as necessary.

In addition, in certain instances, it is expected that the pharmaceutical compositions may be disposed within transdermal devices placed upon, in, or under the skin. Such devices include patches, implants, and injections which release the active ingredient by either passive or active release mechanisms. Transdermal administrations include all administration across the surface of the body and the inner linings of bodily passage including epithelial and mucosal tissues. Such administrations may be carried out using the present compositions in lotions, creams, foams, patches, suspensions, solutions, and suppositories (rectal and vaginal).

Transdermal administration may be accomplished through the use of a transdermal patch containing an active ingredient (*i.e.,* an anti-Claudin-1 antibody, or a biologically active fragment thereof) and a carrier that is non-toxic to the skin, and allows the delivery of the ingredient for systemic absorption into the bloodstream *via* the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may be suitable. A variety of occlusive devices may be used to release the active ingredient into the bloodstream such as a semi-permeable membrane covering a reservoir containing the active ingredient with or without a carrier, or a matrix containing the active ingredient.

Suppository formulations may be made from traditional materials, including cocoa butter, with or without the addition of waxes to alter the suppository's melting point, and glycerine. Water soluble suppository bases, such as polyethylene glycols of various molecular weights, may also be used.

Materials and methods for producing various formulations are known in the art and may be adapted for practicing the subject invention. Suitable formulations for the delivery of antibodies can be found, for example, in *"*Remington's Pharmaceutical Sciences", E.W. Martin, 18th Ed., 1990, Mack Publishing Co.: Easton, PA.

### B. Additional Biologically Active Agents

In certain embodiments, an anti-Claudin-1 antibody, or a biologically active fragment thereof, is the only active ingredient in a pharmaceutical composition of the present invention. In other embodiments, the pharmaceutical composition further comprises one or more biologically active agents. Examples of suitable biologically active agents include, but are not limited to, therapeutic agents such as anti-viral agents, anti-inflammatory agents, immunomodulatory agents, analgesics, antimicrobial agents, kinase inhibitors, signalling inhibitors, antibacterial agents, antibiotics, antioxidants, antiseptic agents, and combinations thereof. Examples of other suitable biologically active agents include the therapeutic agents suitable for the treatment of pulmonary fibrosis and for the treatment of kidney fibrosis, pulmonary fibrosis or skin fibrosis, such as those listed above.

In such pharmaceutical compositions, the anti-Claudin-1 antibody and additional therapeutic agent(s) may be combined in one or more preparations for simultaneous, separate or sequential administration of the anti-Claudin-1 antibody and therapeutic agent(s). More specifically, an inventive composition may be formulated in such a way that the antibody and therapeutic agent(s) can be administered together or independently from each other. For example, an anti-Claudin-1 antibody and a therapeutic agent can be formulated together in a single composition. Alternatively, they may be maintained (*e.g*., in different compositions and/or containers) and administered separately.

### C. Pharmaceutical Packs of Kits

In another aspect, the present invention provides a pharmaceutical pack or kit comprising one or more containers (*e.g*., vials, ampoules, test tubes, flasks or bottles) containing one or more ingredients of an inventive pharmaceutical composition, allowing administration of an anti-Claudin-1 antibody, or a biologically active fragment thereof.

Different ingredients of a pharmaceutical pack or kit may be supplied in a solid (*e.g*., lyophilized) or liquid form. Each ingredient will generally be suitable as aliquoted in its respective container or provided in a concentrated form. Pharmaceutical packs or kits may include media for the reconstitution of lyophilized ingredients. Individual containers of the kits will preferably be maintained in close confinement for commercial sale.

In certain embodiments, a pharmaceutical pack or kit includes one or more additional therapeutic agent(s) as described above. Optionally associated with the container(s) can be a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of a pharmaceutical composition according to methods of treatment disclosed herein.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data are actually obtained.

### Example 1: Anti-Claudin-1 Monoclonal Antibody Biodistribution in vivo Materials and Methods

**Reagents and Antibodies.** The anti-Claudin-1 monoclonal antibody (anti-CLDN1 mAb - OM-7D3-B3) was produced as previously described (Fofana et al., Gastroenterology, 2010, 139: 953-964, e1-4). Control mAb (rat IgG2b clone LTF-25, Bio X Cell) was also used.

**Research Experiment on Live Vertebrates.** *In vivo* experiments were performed in the INSERM Unit 1110 animal facility according to local laws following ethical committee approval (CREMEAS, projet numbers AL/02/19/08/12 and AL/01/18/08/12).

**Antibody biodistribution.** The antibodies were labeled with Alexa-fluor 750 (RD-Biotech, Besançon, France). Eight week-old Balb/c mice were injected intraperitoneally with 500 µg of Alexa-fluor 750-labeled CLDN1-specific or control mAb. Organs were harvested 24 hours and 48 hours as previously described (Krieger et al., Hepatology, 2010, 51: 1144-1157) and *ex vivo* organ-specific fluorescence was detected with an IVIS Lumina 50 (Xenogen-Caliper-Perkin-Elmer) and expressed as average efficiency.

**Statistical Analyses.** The Mann-Whitney test was used. A *p*-value ≤ 0.05 was considered significant. Statistical analyzes were performed with GraphPad Prism 6 software.

### Results

**Anti-Claudin-1 Antibody Biodistribution.** The present Inventors have measured the *in vivo* biodistribution of the anti-CLDNl monoclonal antibody in Balb/c mice and compared it to a control monoclonal antibody. The results presented in Figure 1 show that an enrichment in the anti-CLDNl monoclonal antibody was observed in skin, kidneys, lungs, intestines and liver (see Figure 1(A) measured at 24 hours and Figure 1(B) measured at 48 hours) (Mailly et al., Nature Biotechnol. 2015, 33(5): 549-554).

### Example 2: In vivo Efficacy of an Anti-Claudin-1 Monoclonal Antibody in

### Preventing Fibrosis in a Bleomycin-Induced Pulmonary Fibrosis Model Materials and Methods

**Protocol to Examine the Effects of an anti-human CLDN1 Monoclonal Antibody in Preventing Fibrosis in a Bleomycin-Induced Pulmonary Fibrosis Model.** Pathogen-free 6 weeks old female C57BL/6Jmice were obtained from Japan SLC, Inc. (Japan). At Day 0,54 mice were induced to develop pulmonary fibrosis by a single intratracheal administration of bleomycin hydrochloride (BLM, Nippon Kayaku, Japan) in saline at a dose of 3.0 mg/kg, in a volume of 50 µL per animal using Microsprayer® (Penn-Century, USA). In each slot, the mice were randomized into 3 groups of 18 mice based on their body weight on the day before the start of treatment at Day 0. Individual body weight were measured daily during the experimental period. Survival, clinical signs and behaviour of mice were monitored daily.

***Mice groups.*** Group 1 (Vehicle): Eighteen bleomycin-induced pulmonary fibrosis model mice were intraperitoneally administered vehicle [Saline] in a volume of 5 mg/kg once weekly from Day 0 to 20. Group 2 (Anti CLDN1 Ab): Eighteen bleomycin-induced pulmonary fibrosis model mice were intraperitoneally administered vehicle supplemented with Anti CLDN1 Ab at a dose of 500 µg/mouse once weekly from Day 0 to 20. Group 3 (Dexamethasone): Eighteen bleomycin-induced pulmonary fibrosis model mice were orally administered pure water supplemented with Dexamethasone at a dose of 0.25 mg/kg (in a volume of 10 mL/kg) once daily from Day 0 to 20. Mice in all groups were sacrificed at Day 21.

***Survival, biochemical and histological analysis.*** Mice in all groups were sacrificed at Day 21 for the Survival and histological essays. Survival curves were established using the Kaplan-Meier survival method and compared using the Log Rank Test. Histological analysis for lung sections was performed according to a routine method: Masson's Trichrome staining and estimation of Ashcroft score.

***Statistical tests.*** Statistical tests were performed using Kruskal-Wallis test. P values < 0.05 were considered statistically significant.

The study design is summarized in Figure 2(A).

### Results

The anti-CLDNl mAb group showed a significant decrease in the Ashcroft score compared with the Vehicle group (respective mean±SD were 3.9±1.5 and 2.8±0.9, p<0.05). The Ashcroft score in the Dexamethasone group tended to decrease compared with the Vehicle group (Figure 2(B)). Representative photomicrographs of Masson's Trichrome-stained lung sections are shown in Figure 2(C).

During the treatment period, mice found dead before reaching Day 21 were as follows; four out of 18 mice were found dead in the Vehicle group. Five out of 18 mice were found dead in the Anti-CLDN1 mAb group. Ten out of 18 mice were found dead in the Dexamethasone group. The Dexamethasone group showed a significant decrease in survival rate compared with the Vehicle group. There was no significant difference in survival rate between the Vehicle group and the Anti CLDN1 Ab group (Figure 2(D)).

Body weight was expressed as percentage of body weight change from baseline (Day 0). Mean body weight changes of the Dexamethasone group were significantly lower than that of the Vehicle group at Day 4-16, 18, 19 and 21. There were no significant differences in mean body weight changes at any day during the study period between the Vehicle group and the Anti CLDN1 Ab group (Figure 2(E)).

### Example 3: In vivo Efficacy of an Anti-Claudin-1 Monoclonal Antibody in

### Treating Fibrosis in a Bleomycin-Induced Pulmonary Fibrosis Model Materials and Methods

***Protocol to Examine the Effects of anti-human CLDN1 Monoclonal Antibody in Treating Fibrosis in a Bleomycin-Induced Pulmonary Fibrosis Model.*** Pathogen-free 6 weeks old female C57BL/6Jmice will be obtained from Japan SLC, Inc. (Japan). On Day 0, 54 mice were anesthetized with pentobarbital sodium (Kyoritsu Seiyaku, Japan) and intratracheally administered bleomycin (Lot # 771840, Nippon Kayaku, Japan) in saline at a dose of 3 mg/kg, in a volume of 50 µL per animal using a Microsprayer® (Penn-Century, USA). The mice were transferred to a clean cage (resting cage) and kept until recovery from anesthesia. The bleomycin administration took place on two separate days, with equal numbers of mice assigned to each day. In each slot, bleomycin-induced pulmonary fibrosis model mice were randomized into 3 groups of 18 mice based on their body weight changes on the day before the start of treatment at Day 7. Survival, clinical signs and behaviour of mice were monitored daily.

***Mice groups.*** Group 1 (Vehicle): Eighteen bleomycin-induced pulmonary fibrosis model mice were intraperitoneally administered vehicle [Saline] in a volume of 5 mg/kg once weekly from Day 7 to 20. Group 2 (Anti CLDN1 Ab): Eighteen bleomycin-induced pulmonary fibrosis model mice were intraperitoneally administered vehicle supplemented with Anti CLDN1 Ab at a dose of 500 µg/mouse once weekly from Day 7 to 20. Group 3 (Nintedanib): Eighteen bleomycin-induced pulmonary fibrosis model mice were orally administered pure water supplemented with Nintedanib at a dose of 100 mg/kg (in a volume of 10 mL/kg) once daily from Day 7 to 20. Mice in all groups were sacrificed at Day 21 for the following assays.

***Survival, biochemical and histological analysis.*** Mice in all groups were sacrificed at Day 21 for the Survival and histological essays. Survival curves were established using the Kaplan-Meier survival method and compared using the Log Rank Test. Lung hydroxyproline content was assessed according to a routine method.

***Statistical tests.*** Statistical tests were performed using Kruskal-Wallis test. P values <0.05 will be considered statistically significant.

The study design is summarized in Figure 3(A).

### Results

The anti-CLDN1 mAb group showed a significant decrease in the lung hydroxyproline levels compared with the Vehicle group (respective mean±SD were 52±8.3 and 45.3±8.4, p<0.05). The lung hydroxyproline levels in the Nintedanib group tended to decrease compared with the Vehicle group (mean±SD 51.3±9.0, see Figure 3(B)).

Representative photomicrographs of Masson's Trichrome-stained lung sections are shown in Figure 3(C).

There were no significant differences in survival rate and body weight between the Vehicle group and the treatment groups (see Figure 3(D-E)).

### Example 4: Assessment of an Anti-Claudin-1 Monoclonal Antibody for Therapeutic Applications in Lung and Kidney Fibrosis

To study the therapeutic potential applications of CLDN1-specific mAb for lung and kidney fibrosis, the present inventors have assessed the binding of CLDN1-specific mAb to cells from a kidney cell line (RPTEC/TERT1) and a lung cell line (A549). Furthermore, they have assessed the induction and reversal of the progressive liver signature and TGF-β signalling in A549 lung cells.

### Materials and Methods

***Cell Lines.*** The kidney cancer cell line RPTEC/TERT1 and lung cancer cell line A549, both originally from ATCC, were obtained from IGBMC in a collaboration with Dr. Irwin Davidson and Dr. Izabella Sumara, respectively. Both cell lines were cultured according to provider's instructions: the RPTEC/TERT1 kidney cancer cells were grown in ATCC-formulated DMEM:F12 Medium supplemented with hTERT RPTEC Growth Kit and 0.1 mg/mL G418. The A549 lung cancer cells were grown in ATCC-formulated F-12K Medium supplemented with 10% fetal bovine serum.

***Binding Studies.*** Cells were de-attached using a solution of EDTA (4 mM) and re-suspended in PBS containing 1% FBS at 4°C. Then, 150,000 cells were incubated with increasing concentrations of the humanized H3L3 CLDN1-specific mAb (see WO/2017/162678) and the binding signals were read by flow cytometry after incubation with PE-labelled anti-human using a dilution of 1:100 and analyzed with Cytoflex and FlowJo V10. Two washes with PBS containing 1% FBS were performed after every antibody incubation. A binding curve for each cell line was built using the PE median fluorescence intensity (MFI) and the K_{d} of the interaction was calculated by applying the Michaelis-Menten mathematical model in R 3.5.1.

***PLS Studies.*** A549 cells were seeded on a 12 well plate (30,000 cell/well) and incubated with or without TGF-β (5 ng/mL) (Peprotech) and CLDN1-specific mAb (20 µg/mL) for 24 hours. Subsequently, RNA was extracted using the Promega Reliaprep kit and 125 nm were used to determine the induction of the PLS using nCounter Nanostring.

***TGF-β Signaling Reporter Assay.*** A549 cells were transfected with TGF-β signalling reporter plasmid pGLA4.48[luc2P/SBE/Hygro] (Promega) using Fugene according to the manufacturer's instructions. Following transfection, the cells were supplemented with fresh medium containing control or CLDN1-specific mAb (50 µg/mL) for 3 hours at 37°C. Fresh medium was supplemented to the cells containing TGF-β (10 ng/mL) (Sigma) for 3 hours at 37°C. The plate was incubated for 15 minutes at room temperature followed by addition of ONE-Glo substrate (Promega) for 3 minutes at room temperature. Luminescence of the supernatants was read using a Berthold microplate reader.

### Results

***CLDN1 mAb binds to CLDNA expressed on RPTEC*/*TERT1 kidney and A549 lung cell lines.*** CLDN1 mAb was found to saturate the epitope of both cell lines: 50 µg/mL for the kidney cancer cell line RPTEC/TERT1 (Figure 4(A)) and 20 mg/mL for the lung cancer cell lineA549 (Figure 4(B)), hence resulting in a K_{d} of 53 (Figure 4(C)) and 16.3 nM (Figure 4(D)), respectively. These results confirm that CLDN1-specific mAb is able to bind to CLDN1 expressed by kidney and lung cells.

***CLDN1 mAb reverses fibrosis-related poor-prognosis status of the PLS in A549 lung cell line.*** To further investigate the functional role of CLDN1 as a driver for fibrosis and carcinogenesis, the present inventors have assessed whether CLDN1 mAb modulates the expression of the clinical PLS, a 32-gene signature predictive of fibrosis and liver disease progression, cancer risk and mortality in patients (Hoshida et al., N. Engl. J. Med., 2008, 359: 1995-2004; Hoshida et al., Gastroenterology, 2013, 144: 1024-1030; King et al., Gut, 2015, 64: 1296-1302; Nakagawa et al., Cancer Cell, 2016, 30: 879-890; Goossens et al., Clin. Gastroenterol. Hepatol., 2016, 14: 1619-1628). The A549 cell line is known to induce the clinical PLS without the presence of a stress inducer (FDR = 0.018 for poor prognosis genes and 0.012 for good prognosis genes). Treatment with CLDN1 mAb for 24 hours resulted in the reversal of the clinical PLS (FDR = 0.094 for poor prognosis genes and 0.095 for good prognosis genes) (Figure 5(A)). Two PLS poor prognosis genes (SERPINB2 and FMOl) were significantly down-regulated following CLDN1-specific mAb with fold changes less than 1.5 (Figure 5(B)). These data suggest that CLDN1 acts as a driver for the poor prognosis of the clinical PLS in a lung fibrosis cell line model and support the potential role of CLDN1 mAb as a candidate therapeutic target.

***CLDN1 mAb reduces TGF-b signalling in A549 lung cells.*** TGF-β is essential for the differentiation of lung fibroblasts into myofibroblasts which is a key step in the development of tissue fibrosis. Moreover, increased expression of TGF-β has been reported in fibrotic lungs. TGF-β contributes to the ECM production including collagen, laminin, and fibronectin (Saito et al., Int. J. Mol. Sci., 2018, 19(8): 2460). To further investigate the effect of CLDN1-specific mAb on TGF-β signalling in lung cells, transfected SBE (TGF-β signalling reporter plasmid) A549 cells were treated with TGF-β (10 ng/mL) and CLDN1-specific mAb resulted in significant reduction in TGF-β reporter activity (Figure 5(B)).

### Conclusion

CLDN1-specific mAb binds to CLDN1-expressing cells from kidney (RPTEC/TERT1) and lung (A549) cell lines. Furthermore, it modulates the reversal of PLS, predictive of fibrosis, in A549 cells. This suggests a role of CLDN1-specific mAb in alleviating fibrosis progression. Lastly, it was shown that CLDN1 mAb reduces TGF-β signalling, a crucial pathway for lung fibrosis. Altogether, these data suggest the potential therapeutic effect of CLDN1-specific mAb for lung and kidney fibrosis.

### Example 5: Assessment of an Anti-Claudin-1 Monoclonal Antibody for Therapeutic Applications in Skin Fibrosis - BLM-induced Skin Fibrosis Model

Systemic sclerosis (SSc) is a chronic connective tissue disease of unknown cause. It is characterized by fibrosis of the skin and internal organs. Most patients develop tissue fibrosis and organ dysfunction in the late stages of SSc, which results in increased mortality. Symptomatic treatments of SSc are currently limited, and causal therapies have been long awaited.

Bleomycin (BLM)-induced skin fibrosis model is a well characterized disease model for skin fibrosis and commonly used for studying biological pathways of SSc. The BLM model encompasses key pathophysiological features of SSc: epidermal hypertrophy and dermal fibrosis, which makes this model attractive for a simple proof-of-concept study for SSc, or *in vivo* screening for anti-fibrosis molecules (Yamamoto et al., J. Invest. Dermatol, 1999, 112: 456).

### Materials and Methods

***BLM Model and Sampling.*** As indicated on the scheme presented on Figure 6, the BLM is subcutaneously injected every other day into the pre-shaved back of mice for 4 weeks. The injection sites are located at the corners of a 1.5 cm²-square shaved area and used in rotation (corner 1, corner 2, corner 3, corner 4, corner 1, etc). A 5-mm circle is clipped using a dermal punch and used for collagen quantification. The remaining intact area is cut out into 3 rectangles, one for histology and two for biochemical analyses. HE staining of skin samples from BLM-induced skin fibrosis model shows an increased thickness 28 days after the BLM injections. In parallel, the subcutaneous adipose layer shows atrophy and shrinkage.

***Effects of Imatinib on Skin Fibrosis.*** Imatinib mesylate is a tyrosine kinase inhibitor against c-Abl, PDGFR, and several other tyrosine kinases (Alfiya et al., Arthritis Rheum., 2009, 60: 219). c-Abl is crucial for induction of ECM proteins through the TGF-β pathway. Its inhibition may reduce the synthesis of EMC components. Imatinib interferes with PDGF signalling by blocking the tyrosine kinase activity of PDGFR (Alfiya et al., Arthritis Rheum., 2009, 60: 219). The study design is presented on Figure 7.

### Results

The results obtained are presented on Figure 8. Imatinib was found to induce a significant decrease in dermal thickness and in the skin fibrosis area.

A similar experiment will be carried out using the CLDN1-specific mAb instead of Imatinib.

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. An anti-Claudin-1 antibody, or a biologically active fragment thereof, for use in the prevention or treatment of a fibrotic disease selected from pulmonary fibrosis, renal fibrosis and skin fibrosis, in a subject.

2. The anti-Claudin-1 antibody, or the biologically active fragment thereof, for the use according to claim 1, wherein the pulmonary fibrosis is selected from the group consisting of idiopathic pulmonary fibrosis (IPF), idiopatic nonspecific interstitial pneumonitis (NSIP), cryptogenic organizing pneumonia (COP), Hamman-Rich syndrome, lymphocytic interstitial pneumonitis (LIP), respiratory bronchiolitis interstitial lung disease, desquamative interstitial pneumonitis or idiopathic lymphoid interstitial pneumonia, and idiopathic pleuroparenchymal fibroelastosis.

3. The anti-Claudin-1 antibody, or the biologically active fragment thereof, for the use according to claim 1 or claim 2, wherein the pulmonary fibrosis is associated with chronic obstructive pulmonary disease.

4. The anti-Claudin-1 antibody, or the biologically active fragment thereof, for the use according to any one of claims 1 to 3, wherein the fibrotic disease is pulmonary fibrosis and wherein the anti-Claudin-1 antibody, or the biologically active fragment thereof, is administered in combination with at least one therapeutic agent selected from the group consisting of corticosteroids, anti-fibrotic agents, pirfenidone, nintedanib, and anti-acid drugs, and/or with a therapeutic procedure selected from the group consisting of lung transplantation, hyperbaric oxygen therapy and pulmonary rehabilitation.

5. The anti-Claudin-1 antibody, or the biologically active fragment thereof, for the use according to claim 1, wherein the renal fibrosis is renal interstitial fibrosis or glomerulosclerosis.

6. The anti-Claudin-1 antibody, or the biologically active fragment thereof, for the use according to claim 1 or claim 5, wherein the renal fibrosis is associated with chronic kidney disease.

7. The anti-Claudin-1 antibody, or the biologically active fragment thereof, for the use according to any one of claims 1, 5 and 6, wherein the fibrotic disease is renal fibrosis and wherein the anti-Claudin-1 antibody, or the biologically active fragment thereof, is administered in combination with at least one therapeutic agent selected from the group consisting of anti-hypertensive drugs 1,25-dihydroxyvitamin D3, erythropoietin, angiotensin converting enzyme inhibitors, angiotensin II receptor antagonists AST-120, and calcium polystyrene sulfonate, and/or with one therapeutic procedure selected from the group consisting of dialysis and kidney transplantation.

8. The anti-Claudin-1 antibody, or the biologically active fragment thereof, for the use according to claim 1, wherein the skin fibrosis is associated with a medical condition selected from the group consisting of localized scleroderma, systemic scleroderma, graft-versus-host disease (GVHD), nephrogenic fibrosing dermopathy, mixed connective tissue disease, scleredema, scleromyxedema, eosinophilic fasciitis, chromoblastomycosis, hypertrophic scars and keloids.

9. The anti-Claudin-1 antibody, or the biologically active fragment thereof, for the use according to claim 1 or claim 8, wherein the anti-Claudin-1 antibody, or the biologically active fragment thereof, is administered in combination with at least one therapeutic agent selected from the group consisting methotrexate, mycophenolyate, mofetil, cyclophosphamide, cyclosporine, tocilizumab, rituximab, and fresolimumab and/or with at least one therapeutic procedure.

10. The anti-Claudin-1 antibody, or the biologically active fragment thereof, for the use according any one of claims 1 to 9, wherein the anti-Claudin-1 antibody is a monoclonal antibody.

11. The anti-Claudin-1 antibody, or the biologically active fragment thereof, for the use according to any one of claims 1 to 10, wherein the anti-Claudin-1 antibody is a monoclonal antibody having the same epitope as an anti-Claudin-1 monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

12. The anti-Claudin-1 antibody, or the biologically active fragment thereof, for the use according to claim 11, wherein the epitope is strongly dependent on the conservation of the conserved motif W(30)-GLW(51)-C(54)-C(64) in Claudin-1 first extracellular loop.

13. The anti-Claudin-1 antibody, or the biologically active fragment thereof, for the use according to any one of claims 1 to 10, wherein the anti-Claudin-1 antibody is a monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

14. The anti-Claudin-1 antibody, or the biologically active fragment thereof, for the use according to any one of claims 1 to 12, wherein the anti-Claudin-1 antibody is a monoclonal antibody comprising the six complementary determining regions (CDRs) of an anti-Claudin-1 monoclonal antibody secreted by a hybridoma cell line deposited at the DSMZ on July 29, 2008 under an Accession Number selected from the group consisting of DSM ACC2931, DSM ACC2932, DSM ACC2933, DSM ACC2934, DSM ACC2935, DSM ACC2936, DSM ACC2937, and DSM ACC2938.

15. The anti-Claudin-1 antibody, or the biologically active fragment thereof, for the use according to any one of claim 1 to 12 and 14, wherein the anti-Claudin-1 antibody is humanized.

16. The anti-Claudin-1 antibody, or the biologically active fragment thereof, for the use according to claim 15, wherein the anti-Claudin-1 antibody is a humanized monoclonal antibody comprising all of the six CDRs of monoclonal antibody OM-7D3-B3 secreted by the hybridoma cell line deposited under Accession Number DSM ACC2938, wherein the variable heavy chain of OM-7D3-B3 consists of amino acid sequence SEQ ID NO: 1 and the variable light chain of OM-7D3-B3 consists of amino acid sequence SEQ ID NO: 2, said humanized monoclonal antibody further comprising:
a) at least one antibody variable heavy chain (VH) consisting of the amino acid sequence of SEQ ID NO: 3 or SEQ ID NO: 4 or SEQ ID NO: 5, or
b) at least one antibody variable light chain (VL) consisting of the amino acid sequence of SEQ ID NO: 6 or SEQ ID NO: 7 or SEQ ID NO: 8.

17. The anti-Claudin-1 antibody, or the biologically active fragment thereof, for the use according to claim 16, wherein the anti-Claudin-1 humanized monoclonal antibody comprises:
a) two antibody variable heavy chains (VH), both variable heavy chains consisting of the amino acid sequence of SEQ ID NO: 3 or SEQ ID NO: 4 or SEQ ID NO: 5, or
b) two antibody variable light chains (VL), both variable light chains consisting of the amino acid sequence of SEQ ID NO: 6 or SEQ ID NO: 7 or SEQ ID NO: 8.

18. The anti-Claudin-1 antibody, or the biologically active fragment thereof, for the use according to claim 17, wherein the anti-Claudin-1 humanized monoclonal antibody comprises:
1) two antibody variable heavy chains (VH) consisting of the amino acid sequence SEQ ID NO: 3, and two antibody variable light chains (VL) consisting of the amino acid sequence SEQ ID NO: 6 [*H3L3*]; or
2) two antibody variable heavy chains (VH) consisting of the amino acid sequence SEQ ID NO: 3, and two antibody variable light chains (VL) consisting of the amino acid sequence SEQ ID NO: 7 [*H3L1*]*;* or
3) two antibody variable heavy chains (VH) consisting of the amino acid sequence SEQ ID NO: 3, and two antibody variable light chains (VL) consisting of the amino acid sequence SEQ ID NO: 8 [*H3L2*]; or
4) two antibody variable heavy chains (VH) consisting of the amino acid sequence SEQ ID NO: 4, and two antibody variable light chains (VL) consisting of the amino acid sequence SEQ ID NO: 6 [*H1L3*]*;* or
5) two antibody variable heavy chains (VH) consisting of the amino acid sequence SEQ ID NO: 4, and two antibody variable light chains (VL) consisting of the amino acid sequence SEQ ID NO: 7 [*H1L1*]*;* or
6) two antibody variable heavy chains (VH) consisting of the amino acid sequence SEQ ID NO: 4, and two antibody variable light chains (VL) consisting of the amino acid sequence SEQ ID NO: 8 [*H1L2*]*;* or
7) two antibody variable heavy chains (VH) consisting of the amino acid sequence SEQ ID NO: 5, and two antibody variable light chains (VL) consisting of the amino acid sequence SEQ ID NO: 6 [*H2L3*]; or
8) two antibody variable heavy chains (VH) consisting of the amino acid sequence SEQ ID NO: 5, and two antibody variable light chains (VL) consisting of the amino acid sequence SEQ ID NO: 7 [*H2L1*]*;* or
9) two antibody variable heavy chains (VH) consisting of the amino acid sequence SEQ ID NO: 5, and two antibody variable light chains (VL) consisting of the amino acid sequence SEQ ID NO: 8 [*H2L2*].

19. The anti-Claudin-1 antibody, or the biologically active fragment thereof, for the use according to any one of claims 15 to 18, wherein the humanized antibody is a full antibody having an isotype selected from the group consisting of IgG1, IgG2, IgG3, and IgG4.

20. A pharmaceutical composition comprising an effective amount of an anti-Claudin-1 antibody, or a biologically active fragment thereof, and at least one pharmaceutically acceptable carrier or excipient, for use in the prevention or treatment of a fibrotic disease selected from pulmonary fibrosis, renal fibrosis and skin fibrosis, in a subject.

21. The pharmaceutical composition for the use according to claim 20, wherein the fibrotic disease is as defined in any one of claims 2-9 and/or wherein the anti-Claudin-1 antibody, or a biologically active fragment thereof, is as defined in any one of claims 10-19.


SEQUENCE LISTING

<110>  UNISTRA
       INSERM
       Baumert, Thomas

<120>  Anti-Claudin-1 Monoclonal Antibodies for the Prevention and
       Treatment of Fibrotic Diseases

<130>  BEP180876EP

<160>  8

<170>  PatentIn version 3.5

<210>  1
<211>  119
<212>  PRT
<213>  Artificial Sequence

<220>
<223>  variable heavy chain of OM-7D3-B3

<400>  1

Glu Val Gln Leu Val Glu Ser Gly Gly Gly Leu Val Gln Pro Gly Arg
1               5                   10                  15

Ser Leu Lys Leu Ser Cys Leu Gly Ser Gly Phe Ser Phe Ser Ser Tyr
            20                  25                  30

Gly Met Asn Trp Ile Arg Gln Ala Pro Gly Lys Gly Leu Glu Trp Val
        35                  40                  45

Ala Ser Ile Ser Pro Ser Gly Ser Tyr Phe Tyr Tyr Ala Asp Ser Val
    50                  55                  60

Lys Gly Arg Phe Thr Ile Ser Arg Glu Asn Ala Lys Asn Thr Leu Tyr
65                  70                  75                  80

Leu Gln Met Thr Ser Leu Arg Ala Glu Asp Thr Ala Leu Tyr Tyr Cys
                85                  90                  95

Ala Arg Leu Pro Gly Phe Asn Pro Pro Phe Asp His Trp Gly Gln Gly
            100                 105                 110

Val Val Val Thr Val Ser Ser
        115

<210>  2
<211>  107
<212>  PRT
<213>  Artificial Sequence

<220>

<223>  variable light chain of OM-7D3-B3

<400>  2

Asn Thr Val Met Thr Gln Ser Pro Thr Ser Met Phe Met Ser Val Gly
1               5                   10                  15

Asp Arg Val Thr Met Asn Cys Lys Ala Ser Gln Asn Val Gly Gly Asn
            20                  25                  30

Val Asp Trp Tyr Gln Trp Lys Pro Gly Gln Ser Pro Lys Leu Leu Met
        35                  40                  45

Tyr Gly Ala Ser Asn Arg Tyr Thr Gly Val Pro Asp Arg Phe Arg Gly
    50                  55                  60

Ser Gly Ser Gly Thr Asp Phe Thr Leu Thr Ile Ser Asn Met Gln Thr
65                  70                  75                  80

Glu Asp Leu Ala Val Tyr Tyr Cys Leu Gln Tyr Lys Asn Asn Pro Trp
                85                  90                  95

Thr Phe Gly Gly Gly Thr Lys Val Glu Leu Lys
            100                 105

<210>  3
<211>  119
<212>  PRT
<213>  Artificial Sequence

<220>
<223>  humanized variable heavy chain H3

<400>  3

Gln Val Gln Leu Val Glu Ser Gly Gly Gly Val Val Gln Pro Gly Arg
1               5                   10                  15

Ser Leu Arg Leu Ser Cys Leu Gly Ser Gly Phe Ser Phe Ser Ser Tyr
            20                  25                  30

Gly Met Asn Trp Val Arg Gln Ala Pro Gly Lys Gly Leu Glu Trp Val
        35                  40                  45

Ala Ser Ile Ser Pro Ser Gly Ser Tyr Phe Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Thr Ser Leu Arg Ala Glu Asp Thr Ala Ile Tyr Tyr Cys

85                  90                  95

Ala Arg Leu Pro Gly Phe Asn Pro Pro Phe Asp His Trp Gly Gln Gly
            100                 105                 110

Thr Leu Val Thr Val Ser Ser
        115

<210>  4
<211>  119
<212>  PRT
<213>  Artificial Sequence

<220>
<223>  humanized variable heavy chain H2

<400>  4

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Ser Phe Ser Ser Tyr
            20                  25                  30

Gly Met Asn Trp Val Arg Gln Ala Pro Gly Lys Gly Leu Glu Trp Val
        35                  40                  45

Thr Ser Ile Ser Pro Ser Gly Ser Tyr Phe Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Leu Pro Gly Phe Asn Pro Pro Phe Asp His Trp Gly Gln Gly
            100                 105                 110

Thr Leu Val Thr Val Ser Ser
        115

<210>  5
<211>  119
<212>  PRT
<213>  Artificial Sequence

<220>
<223>  humanized variable heavy chain H1

<400>  5

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Ser Phe Ser Ser Tyr
            20                  25                  30

Gly Met Asn Trp Val Arg Gln Ala Pro Gly Lys Gly Leu Glu Trp Val
        35                  40                  45

Ser Ser Ile Ser Pro Ser Gly Ser Tyr Phe Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Leu Pro Gly Phe Asn Pro Pro Phe Asp His Trp Gly Gln Gly
            100                 105                 110

Thr Leu Val Thr Val Ser Ser
        115

<210>  6
<211>  107
<212>  PRT
<213>  Artificial Sequence

<220>
<223>  humanized variable light chain L3

<400>  6

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Gly Asn
            20                  25                  30

Val Asp Trp Tyr Gln Trp Lys Pro Gly Lys Ala Pro Lys Leu Leu Ile
        35                  40                  45

Tyr Gly Ala Ser Asn Arg Tyr Thr Gly Val Pro Asp Arg Phe Arg Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Leu Gln Tyr Lys Asn Asn Pro Trp

85                  90                  95

Thr Phe Gly Gly Gly Thr Lys Val Glu Ile Lys
            100                 105

<210>  7
<211>  107
<212>  PRT
<213>  Artificial Sequence

<220>
<223>  humanized variable light chain L2

<400>  7

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Gly Asn
            20                  25                  30

Val Asp Trp Tyr Gln Trp Lys Pro Gly Lys Ala Pro Lys Leu Leu Ile
        35                  40                  45

Tyr Gly Ala Ser Asn Arg Tyr Thr Gly Val Pro Ser Arg Phe Arg Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Leu Gln Tyr Lys Asn Asn Pro Trp
                85                  90                  95

Thr Phe Gly Gln Gly Thr Lys Val Glu Ile Lys
            100                 105

<210>  8
<211>  107
<212>  PRT
<213>  Artificial Sequence

<220>
<223>  humanized variable light chain L1

<400>  8

Asp Ile Gln Met Thr Gln Ser Pro Ala Thr Leu Ser Val Ser Pro Gly
1               5                   10                  15

Glu Arg Ala Thr Leu Ser Cys Lys Ala Ser Gln Asn Val Gly Gly Asn
            20                  25                  30

Val Asp Trp Tyr Gln Trp Lys Pro Gly Gln Ala Pro Arg Leu Leu Ile
        35                  40                  45

Tyr Gly Ala Ser Asn Arg Tyr Thr Gly Ile Pro Ala Arg Phe Arg Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Leu Gln Tyr Lys Asn Asn Pro Trp
                85                  90                  95

Thr Phe Gly Gln Gly Thr Lys Val Glu Ile Lys
            100                 105